(19) Europäisches Patentamt

European Patent Office

Office européen des brevets

(11) **EP 2 212 436 B1**

(12) **EUROPEAN PATENT SPECIFICATION**

(45) Date of publication and mention
of the grant of the patent:
**12.04.2017 Bulletin 2017/15**

(51) Int Cl.:
***C12Q 1/68*** *(2006.01)*

(21) Application number: **08846449.0**

(22) Date of filing: **07.11.2008**

(86) International application number:
**PCT/GB2008/003760**

(87) International publication number:
**WO 2009/060210 (14.05.2009 Gazette 2009/20)**

(54) **PREDICTIVE TEST FOR ADULT DOG BODY SIZE**

VORHERSAGETEST FÜR DIE KÖRPERGRÖSSE ERWACHSENER HUNDE

ESSAI PRÉDICTIF DE LA TAILLE CORPORELLE DES CHIENS

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **09.11.2007 GB 0722068
04.04.2008 US 42341 P**

(43) Date of publication of application:
**04.08.2010 Bulletin 2010/31**

(73) Proprietor: **MARS, INCORPORATED
McLean, Virginia 22101-3883 (US)**

(72) Inventors:
• **JONES, Paul Glyn**
**Leicestershire LE14 4RT (GB)**
• **HARRIS, Stephen**
**Leicestershire LE14 4RT (GB)**
• **MARTIN, Alan James**
**Leicestershire LE14 4RT (GB)**

(74) Representative: **Roberts, Joanne Nicola
J A Kemp
14 South Square
Gray's Inn
London WC1R 5JJ (GB)**

(56) References cited:
WO-A-2006/090136       WO-A-2007/031792
WO-A-2008/058013       US-A1- 2004 029 145
US-A1- 2006 147 962

• **SUTTER NATHAN B ET AL: "A single IGF1 allele
is a major determinant of small size in dogs"
SCIENCE, AMERICAN ASSOCIATION FOR THE
ADVANCEMENT OF SCIENCE, US,
WASHINGTON, DC, vol. 316, no. 5821, 6 April 2007
(2007-04-06), pages 112-115, XP002480396 ISSN:
0036-8075**
• **MUELLER JAKOB C: "Linkage disequilibrium for
different scales and applications" BRIEFINGS IN
BIOINFORMATICS, vol. 5, no. 4, December 2004
(2004-12), pages 355-364, XP002514317 ISSN:
1467-5463**
• **ROYAL CANIN: "The growth of large and giant
puppies"[Online] February 2007 (2007-02),
XP002514318 Retrieved from the Internet:
URL:http://www.royalcanin.us/brochures/MAX
l_Large_Breed_Puppy_32_brochure.pdf>
[retrieved on 2009-02-06]**
• **ROYAL CANINE: "The growth of small-breed
puppies"[Online] June 2007 (2007-06),
XP002514319 Retrieved from the Internet:
URL:http://www.royalcanin.us/brochures/MIN
l_Indoor_Puppy_27_brochure.pdf> [retrieved on
2009-02-06]**
• **AFFYMETRIX: "GeneChip Canine Genome 2.0
Array" ANNOUNCEMENT AFFYMETRIX, XX, XX,
1 January 2005 (2005-01-01), XP002480398**
• **CHASE KEVIN ET AL: "Interaction between the X
chromosome and an autosome regulates size
sexual dimorphism in Portuguese Water Dogs"
GENOME RESEARCH, COLD SPRING HARBOR
LABORATORY PRESS, WOODBURY, NY, US, vol.
15, no. 12, 1 December 2005 (2005-12-01), pages
1820-1824, XP002480393 ISSN: 1088-9051**

**(Cont. next page)**

- CHASE KEVIN ET AL: "Genetic basis for systems of skeletal quantitative traits: Principal component analysis of the canid skeleton" PROCEEDINGS OF THE NATIONAL ACADEMY OF SCIENCES OF USA, NATIONAL ACADEMY OF SCIENCE, WASHINGTON, DC.; US, vol. 99, no. 15, 23 July 2002 (2002-07-23), pages 9930-9935, XP002480394 ISSN: 0027-8424
- RINCON G ET AL: "Characterization of variation in the canine suppressor of cytokine signaling-2 (SOCS2) gene" GMR. GENETICS AND MOLECULAR RESEARCH,, vol. 6, no. 1, 1 January 2007 (2007-01-01), pages 144-151, XP002480397 ISSN: 1676-5680
- LARK KARL G ET AL: "Genetic architecture of the dog: sexual size dimorphism and functional morphology" TRENDS IN GENETICS, ELSEVIER SCIENCE PUBLISHERS B.V. AMSTERDAM, NL, vol. 22, no. 10, 1 October 2006 (2006-10-01), pages 537-544, XP002480399 ISSN: 0168-9525
- LINDBLAD-TOH KERSTIN ET AL: "Genome sequence, comparative analysis and haplotype structure of the domestic dog" NATURE, NATURE PUBLISHING GROUP, LONDON, UK, vol. 438, no. 7069, 8 December 2005 (2005-12-08), pages 803-819, XP002429578 ISSN: 0028-0836
- WEEDON M N ET AL: "A common variant of HMGA2 is associated with adult and childhood height in the general population" NATURE GENETICS 200710 US, vol. 39, no. 10, October 2007 (2007-10), pages 1245-1250, XP002523244 ISSN: 1061-4036 1546-1718
- BERG J P: "Pygmy mouse gene mutation protects against obesity" EUROPEAN JOURNAL OF ENDOCRINOLOGY 2000 GB, vol. 143, no. 3, 2000, pages 317-318, XP002523245 ISSN: 0804-4643
- DATABASE SNP [Online] 25 May 2006 (2006-05-25), XP002523246 retrieved from NCBI Database accession no. rs22061032
- DATABASE SNP [Online] 25 May 2006 (2006-05-25), XP002523247 retrieved from NCBI Database accession no. rs22003590
- OLIVER F ET AL: "Regulatory variation at glypican-3 underlies a major growth QTL in mice" PLOS BIOLOGY, PUBLIC LIBRARY OF SCIENCE, US, vol. 3, no. 5, 5 April 2005 (2005-04-05), pages 872-877, XP002402688 ISSN: 1544-9173
- DATABASE SNP [Online] 25 May 2006 (2006-05-25), XP002523248 retrieved from NCBI Database accession no. rs24656617

**Description**

**Field of the invention**

The present invention relates to methods of predicting the size of a dog that will be attained in adulthood.

**Background of the invention**

Dogs have the widest variation in size of any mammalian species. The adult bodyweights of the largest breeds are up to 70 times more than those of the smallest breeds. According to the American Kennel Club, in 2006 the most popular large-breed dogs in the USA were the Labrador Retriever, the German Shepherd Dog and Golden Retrievers; the most popular small-breed dogs were Yorkshire Terriers, Dachshunds and Shih Tzus.

Sutter et al (2007) Science 316: 112-115 discloses that a single IGF1 allele is a major determinant of small size in dogs.

**Summary of the invention**

A genetic test for predicting the size of a dog that will be attained in adulthood has now been developed. The present inventors have discovered single nucleotide polymorphisms (SNPs) that are associated with the size of a dog. The identification of these polymorphisms provides the basis for a predictive test to predict the size that a dog will reach when it becomes an adult by screening for a combination of specific molecular markers. The predictive power of the test can be magnified using models that involve combining the results of typing seven defined SNPs. Furthermore, the model can be refined for mixed breed dogs by determining the breed origin of the SNP markers in the dog. Once the size that a dog will become has been predicted, it is then possible to provide care recommendations to the dog owner or carer, such as appropriate diets, in order to achieve the best quality of life for the dog.

Accordingly, the invention provides a method of predicting the size of a dog that will be attained in adulthood, comprising typing the nucleotides present for the combination of single nucleotide polymorphic (SNP) markers present in the genome of the dog at a positions equivalent to position 201 of the following sequences:

- SEQ ID NO: 58 (BICF235J47583, SNP 3);
- SEQ ID NO: 84 (BICFPJ401056, SNP 4);
- SEQ ID NO: 146 (BICF235J47857, SNP 7);
- SEQ ID NO: 7 (BICFPJ1149345, SNP 1);
- SEQ ID NO: 35 (BICF230J67378, SNP 2);
- SEQ ID NO: 96 (BICF235J20169, SNP 5); and
- SEQ ID NO: 111 (BICF235J29129, SNP 6) and thereby predicting the size of the dog that will be attained in adulthood.

The invention further provides:

- a method of predicting the size of a dog that will be attained in adulthood, the method comprising:

   (a) inputting data of the nucleotides, and optionally the breed origin of the nucleotides, present at said SNP marker positions in the dog's genome to a computer system;
   (b) comparing the data to a computer database, which database comprises information relating to one or more said polymorphisms and their association with the size of a dog in adulthood; and
   (c) predicting on the basis of the comparison the size of the dog that will be attained in adulthood;

- use of said SNP markers present in the genome of a dog for predicting the size that a dog will attain in adulthood.

**Brief description of the sequences**

SEQ ID NO: 1 to 146 set out polynucleotide sequences. However, SEQ ID NO: 1-6, 8-34, 36-57, 59-83, 85-95, 97-110 and 112-145 do not relate to the invention.

**Brief description of the drawings**

Figure 1 illustrates schematically embodiments of functional components arranged to carry out the present invention.

Figure 2 shows predicted log breed weight (BW) versus observed log BW by applying a model of the invention to 65 breeds using the average allele frequency for each SNP per breed and the average BW for each breed.

Figure 3 shows a comparison of the predicted weight of 960 dogs calculated from the actual genotype of the dog compared with the average weight for the breed. Each time that average breed weight is referred to it in this document it should be understood to mean the mid-point in the weight range for that breed as determined by reference to "The Encyclopaedia of the dog" by Bruce Fogel, published by Dorling Kindersley, 2000.

Figure 4 illustrates the testing of a model of the invention on mixed breed dogs. The information from Table 7 is plotted graphically. The actual weight (kg) is plotted against the predicted weight (kg) for the Mixed 48 set.

Figure 5 is a graph of the same results as Figure 4 except that the results for male (squares) and female (diamonds) dogs are distinguished.

Figure 6 is a graph showing the effects of the modification matrix when applied to the Mixed 48 set. The arrows demonstrate the change in predicted weight after application of the modification matrix.

## Detailed description of the invention

The present inventors have discovered SNP markers in the dog genome that are determinative of the size of a dog. The present invention therefore provides a method of predicting the size of a dog that will be attained in adulthood using these SNP markers. The term "size" as used herein means the weight or height of the dog. The "predicted" size means that the result is in the form of an estimated, average or approximate size or in the form of a range of size values. The SNPs that have been discovered to be determinative of size are set out in Table 1.

The present invention provides a method of predicting the size of a dog that will be attained in adulthood, comprising typing the nucleotides present for the combination of SNP markers present in the genome of the dog at a positions equivalent to position 201 of the following sequences:

- SEQ ID NO: 58 (BICF235J47583, SNP 3);
- SEQ ID NO: 84 (BICFPJ401056, SNP 4);
- SEQ ID NO: 146 (BICF235J47857, SNP 7);
- SEQ ID NO: 7 (BICFPJ1149345, SNP 1);
- SEQ ID NO: 35 (BICF230J67378, SNP 2);
- SEQ ID NO: 96 (BICF235J20169, SNP 5); and
- SEQ ID NO: 111 (BICF235J29129, SNP 6).

The phrase "typing the nucleotides present for the combination of SNP markers" means genotyping the SNP markers. The presence or absence of each SNP marker is determined. Typically, the nucleotide present at the same position on both homologous chromosomes will be determined. A dog may therefore be determined to be homozygous for a first allele, heterozygous or homozygous for a second allele of the SNP. In discussions herein, a hypothetical first allele may be designated "A" and a hypothetical second allele may be designated "a". In these discussions therefore, the following genotypes are possible for a SNP marker: AA (homozygous), Aa or aA (heterozygous) and aa (homozygous).

The invention further provides the use of the SNP markers present in the genome of a dog for predicting the size that a dog will attain in adulthood.

Any one of the polymorphic positions as defined herein may be typed directly, in other words by determining the nucleotide present at that position.

The combination of the SNP positions as described herein may be typed to carry out the invention. The nucleotides that are typed are selected from positions equivalent to:

- position 201 of SEQ ID NO: 7 (BICFPJ1149345, SNP 1);
- position 201 of SEQ ID NO: 35 (BICF230J67378, SNP 2);
- position 201 of SEQ ID NO: 58 (BICF235J47583, SNP 3);
- position 201 of SEQ ID NO: 84 (BICFPJ401056, SNP 4);
- position 201 of SEQ ID NO: 96 (BICF235J20169, SNP 5);
- position 201 of SEQ ID NO: 111 (BICF235J29129, SNP 6); and
- position 201 of SEQ ID NO: 146 (BICF235J47857, SNP 7).

Typing the nucleotides present in the genome of the dog at positions equivalent to position 201 in a sequence identified in Table 1 mary mean that the nucleotide present at this position in a sequence corresponding exactly with the sequence identified in Table 1 is typed. However, it will be understood that the exact sequences presented in the SEQ ID NOs shown above will not necessarily be present in the dog to be tested. Typing the nucleotide present may therefore be at position 201 in a sequence identified in Table 1 or at an equivalent or corresponding position in the sequence. The term equivalent as used herein therefore means at or at a position corresponding to position 201. The sequence and thus the position of the SNP could for example vary because of deletions or additions of nucleotides in the genome of the dog. Those skilled in the art will be able to determine a position that corresponds to or is equivalent to position 201 in each of the SEQ ID NOs shown above using for example a computer program such as GAP, BESTFIT, COMPARE, ALIGN, PILEUP or BLAST. The UWGCG Package provides programs including GAP, BESTFIT, COMPARE, ALIGN and PILEUP that can be used to calculate homology or line up sequences (for example used on their default settings). The BLAST algorithm can also be used to compare or line up two sequences, typically on its default settings. Software for performing a BLAST comparison of two sequences is publically available through the National Center for Biotechnology Information (http://www.ncbi.nlm.nih.gov/). This algorithm is further described below. Similar publically available tools for the alignment and comparison of sequences may be found on the European Bioinformatics Institute website (http://www.ebi.ac.uk), for example the ALIGN and CLUSTALW programs.

A suitable model for predicting the size of a dog can be established by genotyping SNPs in Table 1, in samples of dogs of different sizes and correlating the allele frequency for each SNP with the sizes of the dogs. One method of correlating the allele frequency is to determine the heterozygosity/homozygosity of each SNP for each dog sample in a panel of samples from dogs of different sizes, for example by giving an allele score for a homozygote (AA) as 0, for a homozygote for the other allele (aa) as 2 and for a heterozygote (Aa or aA) as 1. An average allele score can then be calculated for dogs of approximately the same size or breed.

An association measure can then be used to identify single SNPs associated with size. One example would be the Pearson's product moment correlation coefficient. The SNPs with the highest correlation coefficient are then suitable for incorporation into a model for predicting the particular size parameter of choice. Preferably, the correlation coefficient is greater than 0.3. More preferably, the correlation coefficient is greater than 0.4, 0.45, 0.5, 0.55, 0.6 or 0.65. Once the most significantly associated single SNPs have been identified then the best combination of these SNPs for predicting size can be identified using stepwise regression algorithms, for example by using a statistics package such as Stepwise. These SNPs can then be placed into a model which considers each SNP in series and in which the effect of each SNP is additive.

The inventors have established a model for using the SNPs of the invention to predict the weight of a dog. It will be appreciated that many different models with varying degrees of predictive power are possible, using any number or combination of the SNPs of the invention for predicting any size parameter such as height or weight.

An example of a model suitable for predicting the weight that a dog will attain is now described. This model utilises 7 of the SNPs from Table 1. These 7 SNPs are also set out in Table 2 together. The model is as follows:

$$E(\log(BW)) = 1.69202 + 0.25244X_1 - 0.165X_2 + 0.29516X_3 + 0.51176X_4 - 0.10618X_5 + 0.26279X_6 - 0.30707X_7$$

where $E(\log(BW))$ is "expected log-body weight in kg" and $X_{1-7}$ represents the SNP score at SNPs 1 to 7. SNP scores are 0, 1, or 2, where 0 represents homozygotes for the first allele (AA), 1 represents heterozygotes (Aa and aA) and 2 represents homozygotes for the second allele (aa). The genotype allocated to each SNP score (0, 1 or 2) is set out in Table 3 for each of the 7 SNPs. In applying this equation to predict the average size for a breed the SNP score for all dogs in that breed are averaged.

The present invention provides a method of predicting the size of a dog that will be attained in adulthood, comprising (i) typing the nucleotides present for SNP markers present in the genome of the dog at a position equivalent to position 201 in Table 1 and (ii) inputting the results from (i) into a model that is predictive of the size of the dog. The genotyping results from step (i) may be provided in the form of genotypic values or SNP scores, where a homozygote for one allele is designated a first value (e.g. 0), a heterozygote is designated a second value (e.g. 1) and a homozygote for the other allele is designated a third value (e.g. 2). The predicted value of size may then be determined by multiplying the genotypic value for each SNP by a constant. The constant for each SNP may be different. The constant for each SNP may be the correlation coefficient for the particular SNP with size. The multiplication of the genotypic value for a SNP by a constant is then added to the multiplication of the genotypic value for a second SNP by a constant. This is repeated for each SNP so that the multiplications of each SNP by a constant are added together. Finally, a further constant may be added. The final result is the expected log-body weight in kg.

In one aspect of the invention therefore, the expected log-body weight in kg of a dog is determined by adding the multiplication of the genotypic value of a SNP marker defined herein by a constant and adding to a second constant.

The dog to be tested may be male or female. One general factor which influences how big a dog will be is its sex. Therefore, in one aspect of the method of the invention the sex of the dog may be determined. Determination may for example be by examination by a vet or by questioning the dog's owner. The results of the SNP genotypic values in the model are then multiplied by a sex specific multiplication factor. This multiplication factor may be applied to any number of the SNPs in the model. For example, it may be applied to 1, 2, 3, 4, 5, 6 or all 7 of the SNPs in Table 2.

In any of the methods of the invention described herein all the 7 SNPs in Table 1 are genotyped.

A dog may be tested by a method of the invention at any age, for example from 0 to 12, 0 to 6, 0 to 5, 0 to 4, 0 to 3, 0 to 2 or 0 to 1 years old. Preferably the dog is tested at as young an age as possible, for example within the first year, first 6 months or first 3 months of its life. The dog is preferably tested before it is known how big the dog is going to grow. The aim is therefore to predict the size of the dog that will be attained in adulthood in order to provide care recommendations suitable for the size of the dog.

The dog to be tested by a method of the present invention may be of any breed. Typically the dog will have genetic inheritance of a breed selected from any of the breeds in Table 3 or 4. Popular breeds of dog may be selected from Boston Terrier, Boxer, Bulldog, Chihuahua, American Cocker Spaniel, Daschund, Dobermann Pinscher, German Shepherd Dog, Golden Retriever, Great Dane, Labrador Retriever, Maltese, Miniature Pinscher, Newfoundland, Parson Russell Terrier, Pekingese, Poodle, Poodle (Miniature), Pug, Rottweiler, Schnauzer (Miniature), Shih Tzu, Yorkshire Terrier. The dog may have genetic breed inheritance of a breed that is susceptible to a disease or condition that is affected by size such as canine hip dysplasia (CHD). Breeds of dog that are susceptible to CHD may be selected from Labrador, Golden retriever, German shepherd dog, Rottweiler and Newfoundland.

The dog may be a mixed or crossbred dog, or a mongrel or out-bred dog. The dog may have at least 25%, at least 50%, or at least 100% of its genome inherited from any pure breed or more preferably from any of the breeds described herein. The dog may be a pure-bred. In one embodiment of the invention, one or both parents of the dog to be tested are or were pure-bred dogs. In another embodiment, one or more grandparents are or were pure-bred dogs. One, two, three or all four of the grandparents of the dog that is tested may be or may have been pure-bred dogs.

The method of the invention is particularly useful for predicting the size of a mixed or crossbred dog, or a mongrel or out-bred dog, as information concerning the size of such a dog is less likely to be available to the dog owner or carer compared with a pure-bred dog.

Example 3 demonstrates the capability of a model of the invention to accurately predict the size of mixed-breed dogs. The model can be further refined as described in Example 4 by using information concerning the breed origin of the individual alleles of the

SNP markers. This is useful because, in certain instances, the same SNP is not always associated with the same gene allele in all breeds. For example, for the IGF1 SNP (BICFPJ40156; SNP4; SEQ ID NO: 84) almost all large dog breeds are homozygous for the "a" allele (or "2"). However, despite being a large breed, Rottweilers almost always have the opposite "A" allele (or "0") more commonly associated with small dog breeds. This may indicate that Rottweilers have the "small" version of IGF1. However it is possible that they have the "large" version of the gene but that sometime in their history the "large" version of the gene has become associated with the SNP that is usually associated with the "small" version of the gene. If this holds true, in circumstances when the IGF1 gene has come from a Rottweiler, the genotype of the IGF1 SNP would be misleading.

The invention therefore provides means of refining a model of the invention for -mixed breed dogs by determining the breed origin of the SNP marker alleles for the dog. If the mixed breed dog contains genetic breed inheritance of a breed that has an atypical allele frequency for one or more of the SNP markers in the model, the model can be refined accordingly to take this into account

In more detail, the breed calls of the individual chromosomes in the mixed breed-dog can be used to inform the model. This involves, in certain circumstances, altering the SNP call that is applied to the model, based on the breed that that SNP is thought to have originated from. To follow the example already discussed, if the IGF1 SNP came from a chromosome determined to have come from a Rottweiler, the genotype result would be modified by substituting the SNP allele that is normally associated with the "large" version of IGF1. To achieve this modification, a conversion matrix can be used which takes the genotyped SNP output and translates it into the modified result for dog breeds where it is believed that the SNP may be associated with the wrong allele.

Table 8 provides an example of a conversion matrix for three atypical dog breeds for the IGF 1 SNP (BICFPJ40156; SNP 4; SEQ 1D NO: 84). Each of these dog breeds show unusual IGF1 SNP results compared to their size. This is clear from the average allele frequency of the IGF1 SNP for the atypical breeds compared with similar sized breeds as shown in columns 2 and 3 of Table 8. Column 4 lists the possible genotypes that could be obtained from a sample from a dog. The genotypes are hypothetical, where "A" represents one allele and "a" represents the other allele. The genotype may be converted into a score, as for example in column S, where 0 represents homogygous for a first allele, 1 represents heterozygous and 2 represents homozygous for the second allele. Column 6 lists the possible predicted alleles of a second breed (i.e. a non-atypical breed) that contributes to the genetic breed background of the dog. The allele of the "atypical" breed may then be determined by subtracting the predicted allele of the second breed from the overall genotyped result (column 7).

The predicted allele of the atypical breed can be modified, based on the average allele frequency of the SNP in similar sized breeds (column 8). The resulting modified genotype comprises the modified allele from the atypical breed and an unmodified allele from the second non-atypical breed (column 9). The modified genotype can then be converted into a genotype score (column 10), which can then be applied to the model formula for predicting size.

In order to apply such a conversion matrix, and in one aspect of the invention therefore, the breed origin of each allele for a SNP genotype is determined. This may be determined by (i) determining the breed origin of the chromosome which comprises each allele for a SNP genotype and (ii) predicting which breed contributed to which allele.

The breed origin of each allele for a SNP genotype may be determined by determining the genetic breed background of the dog, i.e. by determining which breeds contributed to the genetic make-up of the dog. The test may be a genetic test, such as a SNP-based or microsatellite-based marker test. An example of a SNP-based test is the commercially available WISDOM PANEL™ MX mixed-breed test. The test may therefore involve genotyping a sample from the dog with a panel of SNPs which allow the breed signatures of the dog to be determined.

A genome-wide panel of SNPs maybe used to determine the genetic breed background of the dog. Alternatively, it is possible to focus on the chromosome containing the "size" SNP of interest to determine the breed origin of the chromosome, for example, by using a panel of SNPs located on the chromosome of interest.

Once the breeds that contribute to the genetic make-up of the dog have been determined it is then necessary to determine which breed contributed to which allele of the genotype. When the genotyped SNP is homozygous (0 or 2) it is self-evident what the allele that has come from the atypical breed must be. However, when the genotyped SNP is heterozygous it is not clear which of the two breeds that contributed to the formation of the chromosome supplied which alleles of the gene. It is necessary therefore to predict the individual genotypes of the chromosomes that came from the problematic breed and the second breed. This can be achieved by reference to a matrix of average allele frequencies for each breed, for example Table 7.

If according to the matrix, all dogs in the second breed are homozygous for a SNP it is possible to confidently predict the allele of the second breed and, by subtraction, the allele of the problematic breed. If according to the matrix the second breed has an average allele frequency nearer to 1 then it is more difficult to determine which allele comes from which breed. In this instance the allele of the second breed can be assigned using a probability that reflects the average allele frequency in that breed. For example, if the allele frequency of the SNP in the breed is 0.8, then the allele can be assigned as follows: A random number between 0 and 2 is selected (to 3 decimal places), for example 1.654. If this number is smaller than the allele fequency of the SNP in the breed in question then the allele is assigned as 0. If it is larger or equal to the allele frequency, then the allele is assigned as 2. In this case, as 1.654 is larger than the average allele frequency for the breed (0.8), the allele of the second breed would be assigned as 2. The prediction of alleles can be carried out using any known statistical package.

Once the breed origin of each allele in the SNP genotype has been predicted, the genotype can be modified to take into account the origin of one or both alleles being from a breed that is known to be atypical for that SNP. After modifying the SNP results, for example by using a conversion matrix, the prediction model/algorithm of the invention can then be used to make a modified prediction of the size of the dog. To illustrate this principle, the conversion matrix described in Table 8 has been used to modify the results of the dogs that are present in a sample of mixed-breed dogs for the IGF1 SNP (Example 4).

According to one aspect of the invention therefore, the method of predicting the size of a dog that will be attained in adulthood further comprises determining the breed origin of the nucleotides present for SNP markers. The method may comprise determining the breed origin of both alleles of a SNP genotype for any of the SNP markers in Table 1.

The breed origin of the nucleotides present for SNP markers may be determined by genotyping a sample from the dog with a panel of genetic markers, such as SNP markers or microsatellites. The predictive test of the invention may therefore be carried out in conjunction with one or more tests for determining the genetic breed background of the dog. Once the genetic breed background has been determined, SNP genotypes can then be modified, if necessary, to take account of the contributions of one or more breeds that have atypical allele frequencies for the particular SNPs. Once the genotypes have been modified, the genotype scores can be applied to the size prediction model in the manner described above.

The predictive test of the invention may be carried out in conjunction with one or more other other predictive or diagnostic tests such as determining susceptibility to one or more diseases. The test may be used in conjunction with a disease susceptibility test to help improve the accuracy of the disease susceptibility prediction, for conditions where expression of the disease phenotype is influenced by the size of the dog. The aim is therefore to improve information about the likelihood of developing the condition.

The test may also be used in conjunction with a disease susceptibility test as part of a preventative or management regime for the condition. In this case, a positive disease susceptibility result for a condition that is influenced by size drives the use of the size predictive test to allow the management of the dogs growth rate/weight in order to reduce the likelihood of developing disease symptoms.

An example of a disease condition that is influenced by size is canine hip dysplasia (CHD). CHD is a congenital disease that causes the hip joints in affected dogs to grow abnormally. The larger the dog, the more likely the dog is to suffer from symptoms of this disease.

*Detection of polymorphisms*

The detection of polymorphisms according to the invention may comprise contacting a polynucleotide of the dog with a specific binding agent for a polymorphism and determining whether the agent binds to the polynucleotide, wherein binding of the agent indicates the presence of the polymorphism, and lack of binding of the agent indicates the absence of the polymorphism.

The method is generally carried out *in vitro* on a sample from the dog, where the sample contains DNA from the dog. The sample typically comprises a body fluid and/or cells of the dog and may, for example, be obtained using a swab, such as a mouth swab. The sample may be a blood, urine, saliva, skin, cheek cell or hair root sample. The sample is typically processed before the method is carried out, for example DNA extraction may be carried out. The polynucleotide in the sample may be cleaved either physically or chemically, for example using a suitable enzyme. In one embodiment the part of polynucleotide in the sample is copied or amplified, for example by cloning or using a PCR based method prior to detecting the polymorphism.

In the present invention, any one or more methods may comprise determining the presence or absence of one or more polymorphisms in the dog. The polymorphism is typically detected by directly determining the presence of the polymorphic sequence in a polynucleotide of the dog. Such a polynucleotide is typically genomic DNA, mRNA or cDNA. The polymorphism may be detected by any suitable method such as those mentioned below.

A specific binding agent is an agent that binds with preferential or high affinity to the protein or polynucleotide having the polymorphism but does not bind or binds with only low affinity to other polynucleotides or proteins. The specific binding agent may be a probe or primer. The probe may be an oligonucleotide. The probe may be labelled or may be capable of being labelled indirectly. The binding of the probe to the polynucleotide may be used to immobilise either the probe or the polynucleotide.

Generally in the method, a polymorphism can be detected by determining the binding of the agent to the polymorphic polynucleotide of the dog. However in one embodiment the agent is also able to bind the corresponding wild-type sequence, for example by binding the nucleotides which flank the variant position, although the manner of binding to the wild-type sequence will be detectably different to the binding of a polynucleotide containing the polymorphism.

The method may be based on an oligonucleotide ligation assay in which two oligonucleotide probes are used. These probes bind to adjacent areas on the polynucleotide that contains the polymorphism, allowing after binding the two probes to be ligated together by an appropriate ligase enzyme. However the presence of a single mismatch within one of the probes may disrupt binding and ligation. Thus ligated probes will only occur with a polynucleotide that contains the polymorphism, and therefore the detection of the ligated product may be used to determine the presence of the polymorphism.

In one embodiment the probe is used in a heteroduplex analysis based system. In such a system when the probe is bound to polynucleotide sequence containing the polymorphism it forms a heteroduplex at the site where the polymorphism occurs and hence does not form a double strand structure. Such a heteroduplex structure can be detected by the use of a single or double strand specific enzyme. Typically the probe is an RNA probe, the heteroduplex region is cleaved using RNAase H and the polymorphism is detected by detecting the cleavage products.

The method may be based on fluorescent chemical cleavage mismatch analysis which is described for example in PCR Methods and Applications 3, 268-71 (1994) and Proc. Natl. Acad. Sci. 85,4397-4401 (1998).

In one embodiment a PCR primer is used that primes a PCR reaction only if it binds a polynucleotide containing the polymorphism, for example a sequence-specific PCR system, and the presence of the polymorphism may be determined by detecting the PCR product. Preferably the region of the primer that is complementary to the polymorphism is at or near the 3' end of the primer. The presence of the polymorphism may be determined using a fluorescent dye and quenching agent-based PCR assay such as the Taqman PCR detection system.

The presence of the polymorphism may be determined based on the change that the presence of the polymorphism makes to the mobility of the polynucleotide during gel electrophoresis. In the case of a polynucleotide, single-stranded conformation polymorphism (SSCP) or denaturing gradient gel electrophoresis (DDGE) analysis may be used. In another method of detecting the polymorphism, a polynucleotide comprising the polymorphic region is sequenced across the region that contains the polymorphism to determine the presence of the polymorphism.

The presence of the polymorphism may be detected by means of fluorescence resonance energy transfer (FRET). In particular, the polymorphism may be detected by means of a dual hybridisation probe system. This method involves the use of two oligonucleotide probes that are located close to each other and that are complementary to an internal segment of a target polynucleotide of interest, where each of the two probes is labelled with a fluorophore. Any suitable fluorescent label or dye may be used as the fluorophore, such that the emission wavelength of the fluorophore on one probe (the donor) overlaps the excitation wavelength of the fluorophore on the second probe (the acceptor). A typical donor fluorophore is fluorescein (FAM), and typical acceptor fluorophores include Texas red, rhodamine, LC-640, LC-705 and cyanine 5 (Cy5).

In order for fluorescence resonance energy transfer to take place, the two fluorophores need to come into close proximity on hybridisation of both probes to the target. When the donor fluorophore is excited with an appropriate wavelength of light, the emission spectrum energy is transferred to the fluorophore on the acceptor probe resulting in its fluorescence. Therefore, detection of this wavelength of light, during excitation at the wavelength appropriate for the donor fluorophore, indicates hybridisation and close association of the fluorophores on the two probes. Each probe may be labelled with a fluorophore at one end such that the probe located upstream (5') is labelled at its 3' end, and the probe located downstream (3') is labelled at is 5' end. The gap between the two probes when bound to the target sequence may be from 1 to 20 nucleotides, preferably from 1 to 17 nucleotides, more preferably from 1 to 10 nucleotides, such as a gap of 1, 2, 4, 6, 8 or 10 nucleotides.

The first of the two probes may be designed to bind to a conserved sequence of the gene adjacent to a polymorphism and the second probe may be designed to bind to a region including one or more polymorphisms. Polymorphisms within the sequence of the gene targeted by the second probe can be detected by measuring the change in melting temperature caused by the resulting base mismatches. The extent of the change in the melting temperature will be dependent on the number and base types involved in the nucleotide polymorphisms.

Polymorphism typing may also be performed using a primer extension technique. In this technique, the target region surrounding the polymorphic site is copied or amplified for example using PCR. A single base sequencing reaction is then performed using a primer that anneals one base away from the polymorphic site (allele-specific nucleotide incorporation). The primer extension product is then detected to determine the nucleotide present at the polymorphic site. There are several ways in which the extension product can be detected. In one detection method for example, fluorescently labelled dideoxynucleotide terminators are used to stop the extension reaction at the polymorphic site. Alternatively, mass-modified dideoxynucleotide terminators are used and the primer extension products are detected using mass spectrometry. By specifically labelling one or more of the terminators, the sequence of the extended primer, and hence the nucleotide present at the polymorphic site can be deduced. More than one reaction product can be analysed per reaction and consequently the nucleotide present on both homologous chromosomes can be determined if more than one terminator is specifically labelled.

Primers or probes may be used in the detection of any of the SNPs defined herein for use in the prediction of size. Polynucleotides may also be used as primers for primer extension reactions to detect the SNPs defined herein.

Such primers, probes and other polynucleotide fragments will preferably be at least 10, preferably at least 15 or at least 20, for example at least 25, at least 30 or at least 40 nucleotides in length. They will typically be up to 40, 50, 60, 70, 100 or 150 nucleotides in length. Probes and fragments can be longer than 150 nucleotides in length, for example up to 200, 300, 400, 500, 600, 700 nucleotides in length, or even up to a few nucleotides, such as five or ten nucleotides, short of a full length polynucleotide sequence.

Primers and probes for genotyping the SNPs of the invention may be designed using any suitable design software known in the art using the SNP sequences in Tables 1 and 2. Homologues of these polynucleotide sequences would also be suitable for designing primers and probes. Such homologues typically have at least 70% homology, preferably at least 80, 90%, 95%, 97% or 99% homology, for example over a region of at least 15, 20, 30, 100 more contiguous nucleotides. The homology may be calculated on the basis of nucleotide identity (sometimes referred to as "hard homology").

For example the UWGCG Package provides the BESTFIT program that can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence that either match or satisfy some positive-valued threshold score T when aligned with a word of the same length in a database sequence. T is referred to as the neighbourhood word score threshold (Altschul *et al*, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as default a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by at least 1, 2, 5, 10, 20 or more mutations, which may be substitutions, deletions or insertions of nucleotides.

The primers or probes may be present in an isolated or substantially purified form. They may be mixed with carriers or diluents that will not interfere with their intended use and still be regarded as substantially isolated. They may also be in a substantially purified form, in which case they will generally comprise at least 90%, e.g. at least 95%, 98% or 99%, of polynucleotides of the preparation.

*Care recommendations and customised food*

A diet for a dog that has been predicted to attain a particular size can be customised. Such a food may be in the form of, for example, wet pet foods, semi-moist pet foods, dry pet foods and pet treats. Wet pet food generally has a moisture content above 65%. Semi-moist pet food typically has a moisture content between 20-65% and can include humectants and other ingredients to prevent microbial growth. Dry pet food, also called kibble, generally has a moisture content below 20% and its processing typically includes extruding, drying and/or baking in heat. The ingredients of a dry pet food generally include cereal, grains, meats, poultry, fats, vitamins and minerals. The ingredients are typically mixed and put through an extruder/cooker. The product is then typically shaped and dried, and after drying, flavours and fats may be coated or sprayed onto the dry product.

A method of preparing customised food for a dog that has had its future size predicted comprises:

(a) predicting the size of a dog that will be attained in adulthood by a method according to the invention; and
(b) preparing food suitable for the dog, wherein the customised dog food comprises ingredients that are suitable for a dog of the predicted size, and/or does not include ingredients that are not suitable for a dog of the predicted size.

Diets tailored specifically to the size of the dog are available commercially. For example, Royal Canin produces four diets called Mini, Medium, Maxi and Giant. Each diet has the appropriate nutritional and energy specification to ensure that the dog receives the correct balance of nutients and sufficient (but not excessive) calories for its size. Versions of each diet are available to feed at puppy, junior and adult stages. The size predictive test of the invention can be used to ensure that a puppy is placed onto the correct diet (e.g. Mini, Medium, Maxi or Giant) to ensure that its energy and nutritional requirements are met.

The size of the dog also influences its risk from certain conditions which can be countered by the use of an appropriate diet. For example, small dogs are at greater risk from tooth decay which can be countered by the use of sodium polyphosphates in the diet helping to trap calcium and therefore reduce the build up of tartar that leads to tooth decay. Alternatively, large dogs are more prone to suffering joint problems because of their increased weight, therefore diets that include joint protecting substances such as chondroitin are advantagous for large dogs. Thus the use of the size prediction test allows the dog to be placed on a diet which both has the correct energy requirements but also contains additives to counter size specific risk factors for its size.

Care recommendations can be provided to a dog owner, veterinarian or dog carer to enable the management of the dog's weight. The predicted size of the dog established using the test of the invention acts as a guide to the dog owner, veterinarian or dog carer of the size that the dog should become and is therefore a useful tool in managing the weight of a dog and in combating obesity.

Furthermore, as explained above, the size prediction test may be used in conjunction with a disease susceptibility test. The size prediction test may improve the accuracy of disease susceptibility prediction for diseases where expression of the disease phenotype is influenced by the size of the dog. Alternatively, following a positive determination of susceptibility to a disease or condition that is influenced by size, the size prediction test may be useful to allow the management of the dog's growth rate or weight. This will reduce the likelihood of the dog developing disease symptoms. Care recommendations that are provided to the dog owner, veterinarian or carer may therefore relate to growth rate or weight management.

*Bioinformatics*

The sequences of the polymorphisms may be stored in an electronic format, for example in a computer database. Accordingly, the invention provides a database comprising information relating to the polymorphisms in Table 1 and the association of the polymorphisms with size. The database may include further information about the polymorphism, for example the degree of association of the polymorphism with dog size or the breed origin of the alleles.

A database as described herein may be used to predict the size of a dog that will be attained in adulthood. Such a determination may be carried out by electronic means, for example by using a computer system (such as a PC). Typically, the determination will be carried out by inputting genetic data from the dog to a computer system; comparing the genetic data to a database comprising information relating to the polymorphisms in Table 1 and the association of the polymorphisms with size; and on the basis of this comparison, predicting the size of a dog that will be attained in adulthood. Information concerning the breed origin of the alleles of the polymorphism may optionally be inputted to the computer system in order to aid size determination of a mixed-breed dog.

Described is a computer program encoded on a computer-readable medium and comprising program code means which, when executed on a computer system, performs all the steps of:

(a) inputting data of the nucleotides, and optionally the breed origin of the nucleotides, present at the said SNP marker positions in the dog's genome to a computer system;
(b) comparing the data to a computer database, which database comprises information relating to the said polymorphisms and their association with the size of a dog in adulthood; and
(c) predicting on the basis of the comparison the size of the dog that will be attained in adulthood.

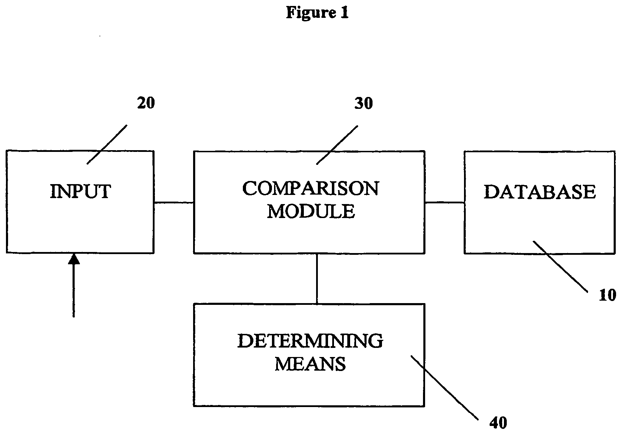

Figure 1 illustrates an apparatus arranged to perform a method according to the invention. The apparatus typically comprises a computer system, such as a PC. In one embodiment, the computer system comprises: means 20 for receiving genetic data from the dog; a module 30 for comparing the data with a database 10 comprising information relating to polymorphisms; and means 40 for predicting on the basis of said comparison the size of a dog that will be attained in adulthood.

The invention is illustrated by the following Examples:

## Example 1

### Methodology

An investigation was conducted to identify SNPs in the canine genome involved in size determination. SNPs were investigated in the 65 dog breeds set out in Tables 4 and 5.

Firstly, for each of the breeds, the average weight and height of the breed was determined using the mid point in the weight or height range for that breed taken from "The Encyclopaedia of the dog" by Bruce Fogel, published by Dorling Kindersley, 2000. Each SNP out of two collections of SNPs (described below) was genotyped in samples of dog genomic DNA for each breed. For each SNP, the genotype in each dog sample was given a designated allele score: a homozygote for one allele was designated as 0, a homozygote for the other allele was designated as 2 and a heterozygote was designated as 1. Then, for each SNP the average allele score per breed was calculated. SNPs that are near to genes that are important for determining breed characteristics tend towards homozygosity, i.e. the average is near to 0 or 2.

To then find which SNPs were best at discriminating size, we grouped the breeds into dogs of a similar size (either height or weight depending on the analysis being performed) and then took the average SNP score across the whole group. Finally, for each SNP we looked for the largest difference in allele score between two groups of dogs of differing average sizes. As an example at the extreme end that meant looking for the SNPs with the largest difference in SNP score between a group of tiny dogs including breeds like Chihuahuas and Yorkshire terriers and another group containing breeds like Great danes and Mastiffs. We then ranked all the SNPs for the difference in SNP score between size groups; those with the largest score were best at separating breed based on size.

### Datasets

Two datasets of SNP genotyping were used for the project:

Dataset 1 comprised data from 3140 dogs genotyped from 87 different breeds at 4608 SNPs. These SNPs are spread out relatively evenly across the genome (with the exception of the sex chromosomes which are not represented).
Dataset 2 comprised data for SNPs selected from regions that were good at distinguishing between breeds in dataset 1. As a result dataset 2 had less SNPs (1536) and these are distributed at a much smaller number of locations on the genome but in greater numbers at each location. In addition the number of samples was increased (4140) and the number of breeds covered was increased dramatically to 163.

To reduce the problem of artificially enhanced homozygosity caused by low sample numbers per breed, dogs from breeds that were represented less than 7 times in the dataset were removed.

**Analysing the data.**

Both datasets were analysed for height and weight. In determining which SNPs were the best to pursue the following criteria were taken into account:

1 SNPs were ranked according to largest difference in allele score between groups.
2 Extra importance was ascribed to locations where several nearby SNPs all scored highly.
3 Extra importance was given to SNPs that were located near to genes known or suspected to be involved in size regulation.

Using these criteria, 102 potentially interesting loci were selected for the new round of genotyping that was to follow. Once we had identified potentially interesting regions of the genome the next step was to genotype further SNPs in these regions to both confirm whether they were significant and also to potentially identify new SNPs more tightly linked to the relevant alleles.

**SNP selection**

Once the locations had been determined, and the number of SNPs chosen, new SNPs were selected from the Can Fam 1 SNP list downloaded from the Broad institute website (http://www.broad.mit.edu/mammals/dog/snp2/).
To select the SNPs the following criteria were applied:

1 SNPs were chosen over a region of 600KB which was centred about either the SNP identified in the data analysis or the middle of the candidate gene.
2 SNPs were spread evenly over this region in close pairs.
3 SNPs with more than 3 N's in the 300 base pairs before or after the SNP were not selected.
4 SNPs were selected in decreasing priority order (depending on the source of the SNP) from the list below:

Priority 1- SNPs from Multiple breeds (other than Boxer, Poodle and wolf).
Priority 2 - SNPs from one breed (other than Boxer, Poodle and wolf).
Priority 3 - SNPs from Boxer and Poodle
Priority 4 - SNPs from Boxer or Poodle only
Priority 5 - SNPs from wolf breeds only.

The reason for this priority order is because it was observed that many of the SNPs identified were from multiple breeds other than boxer and poodle. Firstly this may be because the more breeds a SNP has been identified in the more likely it is actually to be a SNP. Secondly some of these other breeds differ in size from the boxer and therefore SNPs from these breeds could be more likely to be involved in size determination.
An "A" list of 2000 SNPs was selected and a corresponding "B" list was also selected. The A list was sent to Sequenom for analysis, the SNPs that failed design criteria were replaced by corresponding SNPs from the B list.

**Selecting Samples**

Selecting samples was a balance between cost and managing to cover all the different sizes of breeds. To avoid problems with low sample numbers giving artificial homozygosity, breeds where a minimum of 10 samples were available were selected. In total 960 samples were selected from 65 breeds. The list of breeds and sample number selected can be seen in Table 3.

**A model for predicting size.**

Once the genotyping had been completed, to identify SNPs predictive of body size differences, a combination of single and multi-marker analysis were undertaken. The data set consisted of 1,579 SNPs genotyped in a total 960 dogs from $n$ = 65 AKC recognized breeds with an average sample size of 14.8 dogs per breed (range 6 - 25).
Two measures of association were used to identify single SNPs associated with log-transformed average male body weight [log(BW)]. The first is Pearson's product moment correlation coefficient, r, which follows a $t$-distribution with ($n$ - 2) degrees under the null hypothesis of no association between marker frequency and log(BW), assuming the data follow independent normal distributions (testing for a significant Pearson's product moment correlation is equivalent to testing for a significant regression of log(BW) on single marker (SNP) allele frequency). The second measure of association we considered was Kendall's $\tau$ statistic which tests for significant association using the joint distribution of *ranks* of the observations. That is, the observations themselves are not used, but rather their relative ordering (1st, 2nd, 3rd, etc.) for both log(BW) and allele frequency. We can think of this test as measuring whether breeds with high (or low body weight) tend to have high (or low) allele frequencies without regard to the actual values of the average body weight or allele frequencies.

Overall, we found that 302 SNPs out of 1,579 tested (19.2%) were significantly associated with log(BW) at the $\alpha$ = 10% significance level./ A measure of how well individual SNPs predict average body weight differences among breeds is the square of the Pearson's product moment correlation coefficient (equivalent to the $R^2$ statistic in Ordinary Least Squares regression). The SNPs surveyed here showed a range of $R^2$ from 0 to 63 % when considered individually. Since body size is a typical quantitative trait (and, thus, likely to be influenced by many genes acting additively), we sought to improve upon single-marker analyses by searching for combinations of SNPs that yield high $R^2$ when considered together.

The number of distinct regression models for $K$ predictor variables is $2^{k-1}$, meaning that it is computationally impossible to consider all possible combinations of 1,579 SNPs when seeking to predict log(BW) (or even for the subset of SNPs that show strong association at the 10% level). Furthermore, since the number of independent observations available for the regression is the number of breeds in our analysis ($n$ = 65), models with more than 64 SNPs will perfectly fit the data. To overcome these hurdles, we used backwards and forwards stepwise regression algorithms implemented in the R statistics package Stepwise. Briefly, stepwise regression algorithms itevratively build regression models by successively adding or removing variables based on the $t$-statistic of estimated regression coefficients. The terms "backwards" and "forwards" describe whether one begins with the full model and removes terms or begins with the mean model and additively add terms. For our data, both approaches converged to the same solution when the full model contained the 60 most significant singly associated SNPs. The final solution we obtained contained 7 SNPs across 6 chromosomes as well as an intercept term (see Table 2 for the 7 SNPs). The adjusted R-squared for this model was 85.8% indicating a very high predictive ability for log(BW). The final prediction equation is:

$$E(\log(BW)) = 1.69202 + 0.25244X_1 - 0.165X_2 + 0.29516X_3 + 0.51176X_4 - 0.10618X_5 + 0.26279X_6 - 0.30707X_7$$

where E(log(BW)) is "expected log-body weight in kg" and $X_{1-7}$ represents the SNP score at SNPs 1 to 7. SNP scores are either 0,1 ,or 2, where 0 represents homozygotes for allele "A", 1 represents heterozygotes and 2 represents homozygotes for allele "a". The genotype allocated to each SNP score (0, 1 our 2) is set out in Table 2 for each of the 7 SNPs.

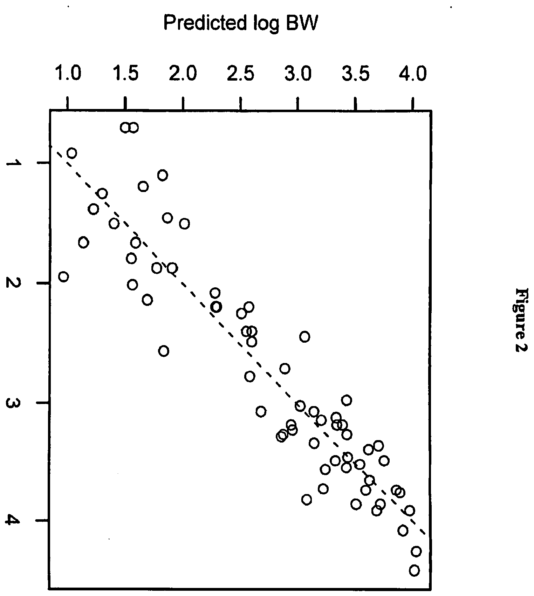

Applying the model to the 65 breeds used in the genotyping, using the average allele frequency per breed, gives the results in Table 4. This is plotted graphically in Figure 2 (BW = Body weight).

## Example 2

### Testing the model

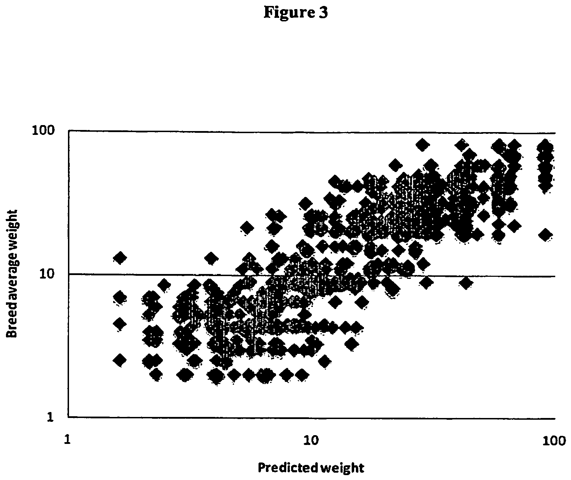

The model determined in Example 1 was tested by using the model to calculate the predicted size of the 960 dogs that went into the size genotyping. In this test we compared the predicted size of the dog calculated from the actual genotype of the dog with the average size for the breed. A good correlation between the predicted and actual weights can be seen from the graph in Figure 3.

We now provide some worked examples that demonstrate how to use the model to predict an individual dog's size:

(1) A dog fixed for all the "small alleles" (i.e., a "0" at all loci with positive effects and a "2" at all loci with negative effects) yields a minimum size of 1.71 kg:

$$\log(y) = 1.69202 + 0(0.25244) + 2(-0.165) + 0(0.29516) + 0(0.51176) + 2(-0.10618) + 0(0.26279) + 2(-0.30707)$$

log(y)= 1.69202 - 0.33 - 0.21236 - 0.61414
y = exp(0.53552)
y = 1.708 Kg

(2) A dog fixed for all the "large alleles" (i.e., a "2" at all loci with positive effects and a "0" at all loci with negative effects) yields a maximum size of 76.43 kg

$$log(y) = 1.69202 + 2(0.25244) + 0(-0.165) + 2(0.29516) + 2(0.51176) + 0(-0.10618)$$
$$+2(0.26279) + 0(-0.30707)$$

log(y)= 1.69202 + 0.50488 + 0.59032 + 1.02352 + 0.52558
y = exp(4.33632)
y = 76.426 Kg

(3) A dog heterozygous for all the size alleles (i.e., a "1" at all the QTLs) yields a size of 11.42633 kg

$$log(y) = 1.69202 + 1(0.25244) + 1(-0.165) + 1(0.29516) + 1(0.51176) + 1(-0.10618)$$
$$+1(0.26279) + 1(-0.30707)$$

log(y)= 1.69202+ 0.25244 - 0.165 + 0.29516 + 0.51176 -0.10618 + 0.26278 - 0.30707
log(y)= 2.43592
y = 11.42633 Kg

(4) A hypothetical dog with some small and large alleles:

$$log(y) = 1.69202 + 2(0.25244) + 2(-0.165) + 2(0.29516) + 2(0.51176) + 2(-0.10618)$$
$$+2(0.26279) + 2(-0.30707)$$

log(y) = 1.69202 + 0.50488 - 0.33 + 0.59032 + 1.02352 - 0.21236 + 0.52558 - 0.61414
log(y)=3.17982
y = 24.04243 Kg

## Example 3

### Validating the model in mixed breed dogs

The model described in Example 2 was generated using data from pure-bred dogs. This Example details the testing of the model on a population of mixed breed dogs.

*Selecting the panel of mixed breed dogs*

The samples used for testing the size model came from a collection of dogs that performed at the "All about dogs" show in the UK The breakdown of the different types of samples collected is provided in Table 5.

Dogs were initially genotyped using the WISDOM PANEL™ MX mixed breed analysis test to confirm they were mixed breed. They were selected for genotyping if their owner considered them mixed breed or if a visual inspection of their photograph suggested they may not be purebred. Of the dogs genotyped, 14 were excluded from the mixed breed set based on the WISDOM PANEL™ MX breed calls. Twelve of these were called as purebreds. Two more, were thought by their owners to be purebreds of breeds outside of the panel (Spanish water dog and American bulldog) and the WISDOM PANEL™ MX result did not contradict this. Once the genotyping with the SNPS from the size model had been performed, a further 4 samples were excluded because they did not genotype for all of the 7 SNPs in the modeL Finally another 14 were excluded because they were not fully grown (i.e. were < 1 year old). This left a set of 48 dogs (called the Mixed 48 set from here on) of which 24 were female and 24 male. Dogs in this set contain no more than 75% of one breed (as determined by WISDOM PANEL™ MX). In all but three cases the dogs contain no more than 50% of one breed. The Mixed 48 set contains 4 dogs that potentially could be Jack Russell terriers. This breed is not in the WISDOM PANEL™ MX test and is also very varied in nature. Photographs of the 4 dogs were studied carefully and although the owners believed them to be Jack Russells, they showed sufficient variability in appearance that they were considered as mixed breed dogs.

*Testing the Size Prediction Model*

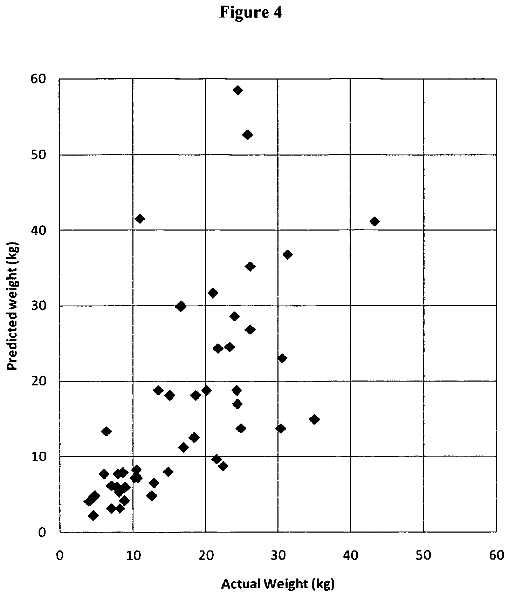

Using the 7 SNP model and the results of the above genotyping analysis, a predicted weight was generated for each dog. Table 6 shows the genotypes for each of the mixed breed dogs along with the predicted weight of the dog and the actual weight of the dog. This information is plotted graphically in Figure 4.

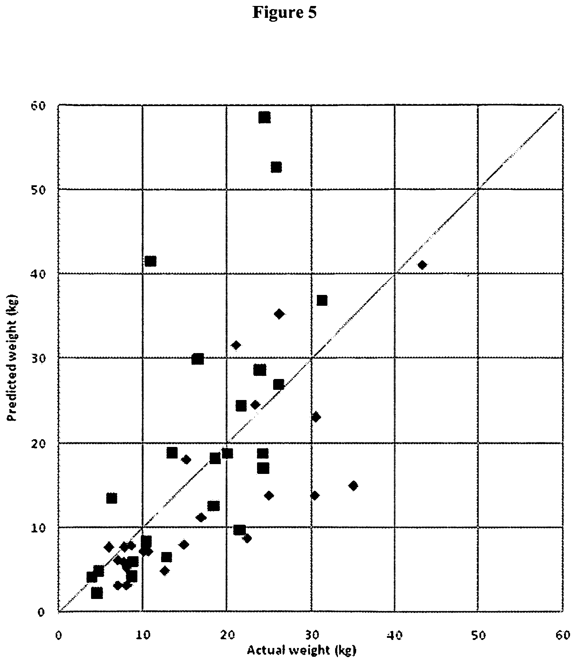

The correlation between the predicted weights and the actual weights of the dogs in this panel is 64% (using the correl function in Excel). This masks the fact that the model is better at predicting the weight of male dogs than female dogs. The predicted weights for the males show a correlation of 78% to the actual weights compared to 64% for the females. This difference in performance between male and female dogs is more obvious when the two sets of data are plotted on the same graph as depicted in Figure 5. The graph shows that the model tends to over predict the weight of some female dogs. This is not surprising given that the model was developed using the weights of male dogs. To refine the model further, it is therefore possible to use information about the sex of the animal to inform the model.

## Example 4

### Refining the model based on the breeds that make up the mixed breed dog.

To demonstrate the effect of taking into account the breed origin of the SNP alleles on the model, the IGF1 SNP was studied. As discussed elsewhere herein, for the IGF1 model SNP (BICFPJ401056) almost all large dog breeds are homozygous for the "2" allele (Table 7). Despite being a large breed, Rottweilers almost all have the opposite allele (0) more commonly associated with small dog breeds. Thus, when the IGF1 gene has come from a Rottweiler, the genotype of the IGF1 SNP would be misleading. The same is true for two other dog breeds considered in this example, namely Bull Terriers and Whippets. In both of these cases, the allele commonly found in these breeds is also opposite to the allele usually found in breeds of a similar size (Table 8).

To take account of this information it was first necessary to develop a modification matrix for each of these three breeds. This can also be seen in Table 8. Once the modification matrix had been developed, the breed origins of chromosome 15 (which contains IGF1) in each dog were then predicted. This was achieved using the WISDOM PANEL™ MX test. The list of chromosome outputs for chromosome 15 for the Mixed 48 set is shown in Table 9. In some cases it was not possible, to unambiguously determine the origins of chromosome 15.

To decide on the correct chromosome outputs, both the predicted best pair of breeds per chromosome and the overall distribution of breed calls for each chromosome was considered (for the selected family tree only). When the predicted best pair of breeds was significantly more likely than the next best pair (i.e.>3 times more likely) then this result was chosen. When the predicted best pair was similar in probability to other pairs of breeds then the overall distribution of breed calls on Chromosome 15 and the other chromosomes was considered in choosing the correct pair. In these cases the choice is somewhat subjective but generally if the probabilities for different pairs of breeds were not very different, preference was given to breeds that appear regularly both on the same chromosome and also on other chromosomes in the same dog. Finally, if applying these criteria had not aided a decision, reference was made to the photograph of the dog and also to the likely probability of that breed being present based on the incidence of the breed.

From Table 9, four dogs were unambiguously determined to contain chromosomes that originate from breeds with atypical IGF1 allele frequencies. These four dogs are highlighted. The results for the IGF1 SNP were then modified according to the matrix in Tablet 8. The SNP results, both before and after modification, are shown in Table 10.

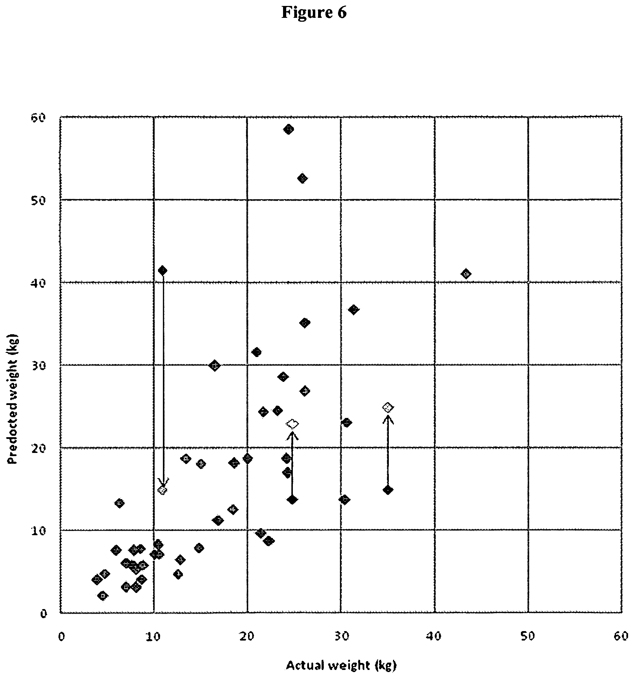

Following this the modified SNP results were then applied to the size precition model. The application of the matrix modifications improves the weight prediction in three of the four cases and in the fourth, applying the modifications has no effect on the result. This is plotted graphically in Figure 6. It is envisaged that a similar procedure could be applied to each of the other 7 SNPs in the model.

Table 1

| SNPs useful for predicting dog size based on correlation of the SNP allele homozygosity score with size (height or weight): |
| :--- |
| For Tables 1 and 2 the Correlation coefficient was calculated using the formula: |

$$Correl(X,Y) \approx \frac{\sum (x-\bar{x})(y-\bar{y})}{\sqrt{\sum (x-\bar{x})^2 \sum (y-\bar{y})^2}}$$

Where X refers to size and Y refers to the SNP score.

| SNP | SEQ ID NO: | Gene Nearby | Chr | Location | Correlation coefficient | Sequence<br>SNP = [wildtype base/alternative base] |
| :--- | :--- | :--- | :--- | :--- | :--- | :--- |
| BICFPJ401056 | 84 | IGF1 | 15 | 44263980 | 0.67387 | CAAGGAAAAGAAGTTATAAACTGGCCCTCTCT AACTTGTACCTGCCTTGCTGTAGGTTGAGGTC TTTCTGAACAATCGTGTCCTTTAGATATCTGG ACCTTCATTAACAGGTTCAGGCTTGGGAACTT GCCAAATTCCAGAAAGGGTCTAGTGAAGGCAT TCAACTGGGGAGCCAGCTGCCTCTTTGGAAAG TGGTTTTA[G/A]TTTACCCTTCATCTTCCAA TAAGAGACAGAATCCCAATTTTCTTAGCTCAA AACCATTTCTTTTAGATTCNAATAGCAAACCT AATGGAACTAATCAACTCAGAGTCCTAAGAAA TAATATTAGAAACTGGCTAAGCATGACAAGGG AAGCAATTTGATATGAGTAAAACACACATTTG TCCACTCAATGCAATTAGAAA |

| SNP | SEQ ID NO: | Gene Nearby | Chr | Location | Correlation coefficient | Sequence<br>SNP = [wildtype base/alternative base] |
|---|---|---|---|---|---|---|
| BICF235J47583 | 58 | HMGA2 | 10 | 11451490 | 0.607791 | AAAAGCANCATATCCAACATTTGTAGTTTGTT<br>ACAATAACACATTGAAAAGATTTATAGACTGT<br>TTTGGGTGTGATTTTTGGATTAATTCCCTACT<br>TTGAAACCATTTGTGAGGCTCTGTTTATTTAA<br>AGGAGGGAATGAATAGACCTGAAAACACCTAA<br>TTTTCATTTTCATCTCAGACTGGAAGCCAGTA<br>CATCTGTA[G/T]GGTTTGTTTTTTGGGTTTT<br>GTTTTGTTTTGTTTTTTTGGTTTTGTTTTGTT<br>TTGTTTAGAATTGAAAACTAGATCACAGAACA<br>CACAATGCTATATTTATCATTTTGATCATCGG<br>TTATTAGATGCTTGTTTGCATGTGCTTAAGCC<br>TCTAGCCAAGATAAAAAAAATTTTNAAAAAC<br>TATTGTGGTAATAGAGTCTAG |
| BICFPJ1149345 | 7 | Growth Hormone receptor | 4 | 70324248 | 0.504142 | ATTGCAATGAATTTGTTTTAATTTGGTGTCTT<br>CACATCCCTGGTTCACCTAGTTACTAACCTGG<br>GGATGTTGTCTCACTCCTCTTGACATAGTGTG<br>TGCCACACAGCAAATGCTCAGTAAGCACTCAC<br>TGAACTGAACTGACTTGCCCAGTACGACTACC<br>AGGGTCAGATTCAACTCACTATAGACTCACTT<br>GCTGACTT[G/T]GATCAAATTTAATTTTATT<br>AAAAATACAAGAACTAGCAGATAGAGGTTGTT<br>GTTGTTGTTTCTAAATCAAACTTATCCTCAGA<br>ACAGTCATTGTAAAAATGATAAATATAGAAGT<br>GTCTCATTTAATAAAAGTTTATGCTATAAAAT<br>CAGTTCTATCGTTAAAAACACCTTAAACATTA<br>GCATCCTCTTTTCCACAGTTT |

EP 2 212 436 B1

| SNP | SEQ ID NO: | Gene Nearby | Chr | Location | Correlation coefficient | Sequence<br>SNP = [wildtype base/alternative base] |
|---|---|---|---|---|---|---|
| BICF235J29129 | 111 | Chr 25 | 25 | 39552390 | 0.472837 | CCACTCATTATGTTCCCTGCAGTATGGAAGTT CTGTGGCCAAGGTTCATATAACTGAGAGTGTA TTTATGGCGGTCCATACTCTTTCTTAGGAAAA TATTGATTTTCTAACAGCAGAATGACTGTAGA GCCGTTAAATCAGACTAGACTATCATAAACTC CAGGATTAACCAAAGAGTACTTTCACCTTTTC TTTTAGTT[A/T]CTCATGAGCCATCGGGAGT AGATACATCCACTTAAGCAGGACAGGATCACA GCATTTATTACTTGATTTGAACAAACCACCAC TATTCCCCACCCTTATTGCCGGATAAGTAATT AAACATTCTGCTCTTATTTTAAAGATTGACTG ACAGGAATGAAAGAGGCCAAGTTGTATTTAAA AAAAAAAAATACAAAGGCTTC |
| BICF235J20169 | 96 | | 20 | 35391970 | 0.382896 | GTTTCCGAGCAGAGATGGAGAAGCAGGGCTTG TAAAATGAACGCCGCCTTCCCCGTTGCATCTT TGCTCCAGGGTGGGGGCCGCCTCGGTTGTAAT TTTACACCGATGTCCACACCCTGCTAGGGAGC AAGAGAGGCGAACTGTAAGTGAGAATATTTGC TCTGCCTCCACCCCCTGGAGGAAGAGGAGCTG GTTCTCTC[G/A]GCAGCCTGCGAGCAGAAGT GGGAGGGCTCCCCCCACCCCAGCCCCTGCGGC CAAGGGCCTGGGGCCATGTGGGTGGGTCCCGA GGAGCAGGTCTTCCCCCCAAAGAGGTGACAAA GACAATGGCAGTTTGAAGGCGCAGCCAGCCCT GCCTTGAGGTAAGGTTGGGGGGTGCCGGTAAGC AGGCTGCTCCGAGAAGGCACC |

EP 2 212 436 B1

18

| SNP | SEQ ID NO: | Gene Nearby | Chr | Location | Correction coefficient | Sequence<br>SNP = [wildtype base/alternative base] |
|---|---|---|---|---|---|---|
| BICF235J47857 | 146 | GLYPICAN 3 | X | 107955905 | 0.5263239 | CTTCACTAGAGTATGAGAACCATGAAGACAGG GACTTTGTTTTGTTCATACTGATTCTCTAGCA CTAAGAGAGCACCTGGCACATGATGCTCAGTA AACATTCCTGGAAGGGGGGAGGGAGGAGGAAG TTTACTATTTCTATATACTAAACACTATGATT TCTGAGTTTGTCTTTTGCCTTTTAAGATTTTT TTTTATTT[A/G]CGTATTTGAGGGGCTCCTG GGTGGCTGGCTCTGTGGTTAGGCGTCTGCCTT CGGCTCAGCATGTGATCCCAGGCCCAGGGATC GAGTCCCGTATTGGGCTCCCTGAGAGGGGCCT GCTTTTCTCTCTGTGTCTCTGCCTCTTTCTGT GTGTCTTTCATGAATAAATTTTTGTTTTAAAA AAGGATTTATTTATTTGAGAG |
| BICF230J67378 | 35 | HMGA2 | 10 | 8445140 | 0.48067147 | CATTACTGGTAATTGTGACCCACTTTTATTTA TCCATTCATTTCACCATTTTTCATAATATAAG TAGGAACCATGAATCTCCTCACCCAAAAGAAG TCAGAACACTCTGATCACAGCTCACATTCAGC TACGTGGTTACTTCCTAGGACATCCCTTTTGA TTCCAGACCTGAGACAATAACCACATTGCCTT CTACATTC[G/A]TAATTCCCTTGATAATCTC GTTATACAGGATTACATCTCCCTATCATTAAG AAATATTTTAGTCATTTTTAACTTTATAAAAA TGGCGTTGCAAATTATTTTTCAGAACTTGTTT TTTACTTAGTATTGTATTGCTAATACTCATTC ATATTTATAAATGCTGTACTTCATTCAACTAC TGTGTCATATTTTATTACTGA |

EP 2 212 436 B1

Table 2

The 7 SNPs used in a model of predicting dog size:

| SNP | Chr | Location | Gene | SNP score 0 | SNP score 1 | SNP score 2 | Sequence SNP = [wildtype base/alternative base] |
|---|---|---|---|---|---|---|---|
| BICFPJ1149345 (SNP 1; SEQ ID NO: 7) | 4 | 70324248 | Growth Hormone receptor | T | TG | G | ATTGCAATGAATTGTTTTAATTTGGTGTC TTCACATCCCTGGTTCACCTAGTTACTAAC CTGGGGATGTTGTCTCACTCCTCTTGACAT AGTGTGTGCCACACAGCAAATGCTCAGTAA GCACTCACTGAACTGAACTGACTTGCCCAG TACGACTACCAGGGTCAGATTCAACTCACT ATAGACTCACTTGCTGACTT[G/T]GATCA AATTTAATTTTATTAAAAATACAAGAACTA GCAGATAGAGGTTGTTGTTGTTTCTAA ATCAAACTTATCCTCAGAACAGTCATTGTA AAAATGATAAATATAGAAGTGTCTCATTTA ATAAAAGTTTATGCTATAAAATCAGTTCTA TCGTTAAAAACACCTTAAACATTAGCATCC TCTTTTCCACAGTTT |
| BICF230J67378 (SNP 2; SEQ ID NO: 35) | 10 | 8445140 | HMGA2 | A | AG | G | CATTACTGGTAATTGTGACCCACTTTTATT TATCCATTCATTTCACCATTTTTCATAATA TAAGTAGGAACCATGAATCTCCTCACCCAA AAGAAGTCAGAACACTCTGATCACAGCTCA CATTCAGCTACGTGGTTACTTCCTAGGACA TCCCTTTGATTCCAGACCTGAGACAATAA CCACATTGCCTTCTACATTC[G/A]TAATT CCCTTGATAATCTCGTTATACAGGATTACA TCTCCCTATCATTAAGAAATATTTTAGTCA TTTTAACTTTATAAAAATGGCGTTGCAAA TTATTTTCAGAACTTGTTTTTTACTTAGT ATTGTATTGCTAATACTCATTCATTATTAT AAATGCTGACTTCATTCAACTACTACTGTGTC ATATTTATTTACTGA |

| SNP | Chr | Location | Gene | SNP score 0 | SNP score 1 | SNP score 2 | Sequence<br>SNP = [wildtype base/alternative base] |
|---|---|---|---|---|---|---|---|
| BICF235J47583 (SNP 3; SEQ ID NO: 58) | 10 | 11451490 | HMGA2 | T | TG | G | AAAAGCANCATATCCAACATTTGTAGTTTG TTACAATAACACATTGAAAAGATTTATAGA CTGTTTTGGGTGTGATTTTTGGATTAATTC CCTACTTTGAAACCATTTGTGAGGCTCTGT TTATTTAAAGGAGGGAATGAATAGACCTGA AAACACCTAATTTTCATTTTCATCTCAGAC TGGAAGCCAGTACATCTGTA[G/T]GGTTT GTTTTTGGGTTTTGTTTTGTTTTGTTTTT TTGGTTTTGTTTTGTTTTGTTTAGAATTGA AAACTAGATCACAGAACACACAATGCTATA TTTATCATTTTGATCATCGGTTATTAGATG CTTGTTTGCATGTGCTTAAGCCTCTAGCCA AGATAAAAAAAATTTTNAAAAACTATTGT GGTAATAGAGTCTAG |
| BICFPJ401056 (SNP 4; SEQ ID NO: 84) | 15 | 44263980 | IGF1 | A | AG | G | CAAGGAAAAGAAGTTATAAACTGGCCCTCT CTAACTTGTACCTGCCTTGCTGTAGGTTGA GGTCTTTCTGAACAATCGTGTCCTTTAGAT ATCTGGACCTTCATTAACAGGTTCAGGCTT GGGAACTTGCCAAATTCCAGAAAGGGTCTA GTGAAGGCATTCAACTGGGGAGCCAGCTGC CTCTTTGGAAAGTGGTTTTA[G/A]TTTAC CCTTCATCTTCCAATAAGAGACAGAATCCC AATTTTCTTAGCTCAAAACCATTTCTTTTA GATTCNAATAGCAAACCTAATGGAACTAAT CAACTCAGAGTCCTAAGAAATAATATTAGA AACTGGCTAAGCATGACAAGGGAAGCAATT TGATATGAGTAAAACACACATTTGTCCACT CAATGCAATTAGAAA |

| SNP | Chr | Location | Gene | SNP score 0 | SNP score 1 | SNP score 2 | Sequence<br>SNP = [wildtype base/alternative base] |
|---|---|---|---|---|---|---|---|
| BICF235J20169 (SNP 5; SEQ m NO: 96) | 20 | 35391970 | ? | A | AG | G | GTTTCCGAGCAGAGATGGAGAAGCAGGGCT<br>TGTAAAATGAACGCCGCCTTCCCCGTTGCA<br>TCTTTGCTCCAGGGTGGGGGCCGCCTCGGT<br>TGTAATTTTACACCGATGTCCACACCCTGC<br>TAGGGAGCAAGAGAGGCGAACTGTAAGTGA<br>GAATATTTGCTCTGCCTCCACCCCCTGGAG<br>GAAGAGGAGCTGGTTCTCTC[G/A]GCAGC<br>CTGCGAGCAGAAGTGGGAGGGCTCCCCCCA<br>CCCCAGCCCCTGCGGCCAAGGGCCTGGGGC<br>CATGTGGGTGGGTCCCGAGGAGCAGGTCTT<br>CCCCCCAAAGAGGTGACAAAGACAATGGCA<br>GTTTGAAGGCGCAGCCAGCCCTGCCTTGAG<br>GTAAGGTTGGGGGTGCCGGTAAGCAGGCTG<br>CTCCGAGAAGGCACC |
| BICF235J29129 (SNP 6; SEQ ID NO:111) | 25 | 39552390 | ? | A | AT | T | CCACTCATTATGTTCCCTGCAGTATGGAAG<br>TTCTGTGGCCAAGGTTCATATAACTGAGAG<br>TGTATTTATGGCGGTCCATACTCTTTCTTA<br>GGAAAATATTGATTTTCTAACAGCAGAATG<br>ACTGTAGAGCCGTTAAATCAGACTAGACTA<br>TCATAAACTCCAGGATTAACCAAAGAGTAC<br>TTTCACCTTTTCTTTTAGTT[A/T]CTCAT<br>GAGCCATCGGGAGTAGATACATCCACTTAA<br>GCAGGACAGGATCACAGCATTTATTACTTG<br>ATTTGAACAAACCACCACTATTCCCCACCC<br>TTATTGCCGGATAAGTAATTAAACATTCTG<br>CTCTTATTTTAAAGATTGACTGACAGGAAT<br>GAAAGAGGCCAAGTTGTATTTAAAAAAAAA<br>AAATACAAAGGCTTC |

EP 2 212 436 B1

22

| SNP | Chr | Location | Gene | SNP score 0 | SNP score 1 | SNP score 2 | Sequence<br>SNP = [wildtype base/alternative base] |
|---|---|---|---|---|---|---|---|
| BICF235J47857 (SNP 7; SEQ ID NO: 146) | X | 107955905 | Glypican 3 | A | AG | G | CTTCACTAGAGTATGAGAACCATGAAGACAGGGACTTTGTTTTGTTCATACTGATTCTCTAGCACTAAGAGAGCACCTGGCACATGATGCTCAGTAAACATTCCTGGAAGGGGGGAGGGAGGAGGAAGTTTACTATTTCTATATACTAAACACTATGATTTCTGAGTTTGTCTTTTGCCTTTTAAGATTTTTTTTTATTT[A/G]CGTATTTGAGGGGCTCCTGGGTGGCTGGCTCTGTGGTTAGGCGTCTGCCTTCGGCTCAGCATGTG ATCCCAGGCCCAGGGATCGAGTCCCGTATTGGGCTCCCTGAGAGGGGCCTGCTTTTCTCTCTGTGTCTCTGCCTCTTTCTGTGTGTCTTTCATGAATAAATTTTTGTTTTAAAAAAGGATTTATTTATTTGAGAG |

**Table 3**

| Breeds of dog and number of samples genotyped | |
|---|---|
| **Breed** | **Number** |
| Afghan Hound | 14 |
| Airedale Terrier | 15 |
| Akita | 15 |
| Alaskan Malamute | 14 |
| American Cocker Spaniel | 15 |
| Basset Hound | 17 |
| Bassett Griff Von Petit | 13 |
| Beagle | 15 |
| Belgian Sheepdog | 13 |
| Bernese Mountain Dog | 24 |
| Bloodhound | 15 |
| Borzoi | 13 |
| Boston Terrier | 14 |
| Boxer | 15 |
| Bull Terrier | 13 |
| Bulldog | 15 |
| Bullmastiff | 20 |
| Chihuahua | 10 |
| Chihuahua (long coat) | 6 |
| Clumber Spaniel | 20 |
| Collie (rough) | 10 |
| Collie (smooth) | 9 |
| Dachshund LH | 14 |
| Dachshund SH | 16 |
| Dachshund WH | 13 |
| Dandie Dinmont Terrier | 6 |
| Dobermann Pinscher | 15 |
| English Springer Spaniel | 15 |
| French Bulldog | 15 |
| German Shepherd Dog | 15 |
| Golden Retriever | 13 |
| Great Dane | 19 |
| Irish Wolfhound | 20 |
| Italian Greyhound | 12 |
| Italian Spinone | 15 |
| Japanese Chin | 10 |

(continued)

| Breeds of dog and number of samples genotyped | |
|---|---|
| **Breed** | **Number** |
| Labrador Retriever | 10 |
| Maltese | 15 |
| Manchester Terrier | 11 |
| Manchester Terrier (toy) | 15 |
| Mastiff | 17 |
| Miniature Pinscher | 21 |
| newfoundland | 25 |
| Norfolk Terrier | 12 |
| Norwich Terrier | 12 |
| Papillon | 20 |
| Parson Russell Terrier, | 15 |
| Pekingese | 7 |
| Pembroke Welsh Corgi | 19 |
| Poodle (Standard) | 17 |
| Poodle Miniature | 10 |
| Portuguese Water Dog | 17 |
| Pug | 15 |
| Rhodesian Ridgeback | 15 |
| Rottweiler | 25 |
| Saint Bernard | 15 |
| Saluki | 20 |
| Samoyed | 15 |
| Schnauzer (Giant) | 17 |
| Schnauzer (Miniature) | 16 |
| Schnauzer (Standard) | 12 |
| Shih Tzu | 10 |
| Siberian Huskey | 19 |
| West Highland white | 11 |
| Yorkshire Terrier | 13 |

**Table 4**

| Applying the model to the 65 breeds | | |
|---|---|---|
| **Breed** | **Predicted weight (kg)** | **Breed average (kg)** |
| Afghan Hound | 20.55 | 25 |
| Airedale terrier | 24.85 | 21.5 |
| Akin | 44.42 | 42 |

(continued)

| Applying the model to the 65 breeds | | |
|---|---|---|
| **Breed** | **Predicted weight (kg)** | **Breed average (kg)** |
| Alaskan Malamute | 35.03 | 47.5 |
| American Cocker Spaniel | 13.88 | 12 |
| Basset Hound | 29.67 | 22.5 |
| Bassett Griffon ven deen (Petit) | 12.98 | 16 |
| Beagle | 12.89 | 11 |
| Belgian Sheepdog | 22.12 | 28 |
| Bernese Mountain Dog | 32.63 | 42 |
| Bloodhound | 55.54 | 43 |
| Borzoi | 27.06 | 41.5 |
| Boston Terrier | 10.55 | 8 |
| Boxer | 37.81 | 28.5 |
| Bull Terrier | 27.55 | 26 |
| Bulldog | 22.60 | 24 |
| Bullmastiff | 52.53 | 50 |
| Chihuahua | 4.60 | 2 |
| Chihuahua (long coat) | 5.16 | 2 |
| Clumber Spaniel | 31.42 | 32.5 |
| Collie (rough) | 27.60 | 24 |
| Collie (smooth) | 26.32 | 24 |
| Dachshund LH | 10.47 | 9 |
| Dachshund SH | 10.02 | 9 |
| Dachshund WH | 12.68 | 9 |
| Dandie Dinmont Terrier | 12.18 | 9.5 |
| Dobermann Pinscher | 24.71 | 35 |
| English Springer Spaniel | 22.96 | 23 |
| French Bulldog | 19.75 | 11.5 |
| German Shepherd Dog | 34.22 | 38.5 |
| Golden Retriever | 30.74 | 31.5 |
| Great Dane | 51.36 | 50 |
| Irish Wolfhound | 37.73 | 47.5 |
| Italian Greyhound | 5.62 | 3.3 |
| Italian Spinone | 39.06 | 32.5 |
| Japanese Chin | 3.74 | 3.5 |
| Labrador Retriever | 33.96 | 29.5 |
| Maltese | 3.19 | 2.5 |
| Manchester Terrier | 5.43 | 7.5 |
| Manchester Terrier (toy) | 3.22 | 4 |

(continued)

| Applying the model to the 65 breeds | | |
|---|---|---|
| **Breed** | **Predicted weight (kg)** | **Breed average (kg)** |
| Mastiff | 70.40 | 82.5 |
| Miniature Pinscher | 7.19 | 4.5 |
| Newfoundland | 53.09 | 59 |
| Norfolk Terrier | 4.78 | 5.3 |
| Norwich Terrier | 3.04 | 5.3 |
| Papillon | 6.61 | 4.3 |
| Parson Russell Terrier | 5.56 | 6.5 |
| Pekingese | 3.89 | 4.5 |
| Pembroke Welsh Corgi | 12.62 | 11 |
| Poodle (Standard) | 17.34 | 26 |
| Poodle Miniature | 5.80 | 13 |
| Portuguese Water Dog | 19.88 | 20.5 |
| pug | 2.78 | 7 |
| Rhodesian Ridgeback | 28.31 | 34.5 |
| Rottweiler | 24.31 | 45.5 |
| Saint Bernard | 73.31 | 70.5 |
| Saluki | 30.27 | 19.5 |
| Samoyed | 18.00 | 26.5 |
| Schnauzer (Giant) | 31.90 | 33.5 |
| Schnauzer (Miniature) | 6.68 | 6.5 |
| Schnauzer (Standard) | 17.27 | 15 |
| Shih Tzu | 4.37 | 6 |
| Siberian Huskey | 14.61 | 21.5 |
| West Highland white terrier | 6.09 | 8.5 |
| Yorkshire Terrier | 5.96 | 3 |

**Table 5**

| A breakdown of the samples used for the size model validation (Example 3) | |
|---|---|
| | No. of samples |
| Genotype | 80 |
| Called as Mixed breed dogs | 66 |
| Mixed breed with all 7 Model SNPs | 62 |
| Mixed mature dogs | 48 |
| Male Mature | 24 |
| Female Mature | 24 |

**Table 6**

| Genotype and size prediction results for the Mixed 48 set (Example 3) | | | | | | | | | | | |
|---|---|---|---|---|---|---|---|---|---|---|---|
| Chr | 4 | 10 | 10 | 15 | 20 | 25 | X | | | | |
| SNP ID | 1 | 2 | 3 | 4 | 5 | 6 | 7 | Predicted (kg) | Actual (kg) | Sex | Age (years) |
| 40051462 | 2 | 2 | 0 | 0 | 1 | 1 | 2 | 4.11 | 3.96 | F | 3 |
| 40047491 | 1 | 2 | 0 | 0 | 2 | 0 | 2 | 2.20 | 4.54 | F | 3 |
| 40049974 | 2 | 2 | 0 | 0 | 2 | 2 | 2 | 4.81 | 4.72 | F | 4 |
| 40043711 | 1 | 2 | 0 | 0 | 1 | 2 | 0 | 7.65 | 596 | M | 2 |
| 40060406 | 2 | 0 | 1 | 1 | 1 | 0 | 1 | 13.39 | 63 | F | 4 |
| 40053199 | 2 | 2 | 0 | 0 | 1 | 0 | 2 | 3.16 | 6.98 | M | 3 |
| 40050815 | 2 | 2 | 0 | 1 | 2 | 1 | 2 | 6.16 | 7.04 | M | 4 |
| 40036652 | 1 | 2 | 0 | 0 | 1 | 1 | 0 | 5.89 | 7.64 | M | 4 |
| 40036518 | 1 | 1 | 1 | 0 | 2 | 2 | 2 | 5.90 | 7.8 | M | 1 |
| 40056463 | 1 | 2 | 0 | 0 | 1 | 2 | 0 | 7.66 | 7.84 | M | 11.75 |
| 40055628 | 1 | 2 | 0 | 0 | 2 | 1 | 0 | 5.30 | 8.08 | M | 3 |
| 40055160 | 2 | 2 | 0 | 0 | 1 | 0 | 2 | 3.16 | 8.14 | M | 4.60 |
| 40048130 | 2 | 2 | 1 | 0 | 1 | 0 | 0 | 7.84 | 8.58 | M | 1.08 |
| 40049353 | 2 | 1 | 0 | 0 | 0 | 0 | 2 | 4.14 | 8.72 | F | 1 |
| 40055850 | 1 | 2 | 0 | 0 | 1 | 1 | 0 | 5.89 | 8.84 | F | 8 |
| 40043243 | 2 | 2 | 1 | 0 | 1 | 2 | 2 | 7.18 | 10.1 | M | 2 |
| 40054384 | 1 | 2 | 1 | 1 | 0 | 0 | 1 | 8.30 | 10.46 | F | 1.6 |
| 40050208 | 2 | 2 | 1 | 0 | 1 | 2 | 2 | 7.18 | 10.6 | M | 2 |
| 40049961 | 2 | 0 | 2 | 2 | 1 0 | 2 | 2 | 41.52 | 10.96 | F | 1.88 |
| 40049466 | 2 | 1 | 0 | 0 | 1 | 1 | 2 | 4.85 | 12.64 | M | 4 |
| 40047766 | 2 | 2 | 1 | 0 | 2 | 2 | 2 | 6.46 | 12.88 | F | 1 |
| 40048252 | 2 | 2 | 1 | 2 | 2 | 1 | 1 | 18.78 | 13.5 | F | 5 |
| 40059930 | 2 | 2 | 1 | 0 | 0 | 2 | 2 | 7.98 | 14.84 | M | 4 |
| 40046359 | 2 | 1 | 1 | 1 | 1 2 | 1 | 0 | 18.05 | 15.1 | M | 9 |
| 40052662 | 2 | 2 | 2 | 2 | 0 | 2 | 2 | 29.85 | 16.54 | F | 4 |
| 40047873 | 2 | 1 | 2 | 1 | 0 | 1 | 0 | 29.98 | 16.62 | F | 2 |
| 40058848 | 2 | 1 | 2 | I | 1 | 0 | 2 | 11.22 | 16.96 | M | 2 |
| 40059059 | 2 | 2 | 1 | 1 | 1 | 1 | 1 | 12.52 | 18.42 | F | 6 |
| 40040572 | 1 | 0 | 2 | 1 | 1 | 1 | 1 | 18.14 | 18.64 | F | 6 |
| 40056389 | 2 | 2 | 2 | 1 | 0 | 1 | 1 | 18.70 | 20.05 | F | 1.6 |
| 40046997 | 2 | 1 | 2 | 2 | 1 | 2 | 2 | 31.66 | 21 | M | 10 |
| 40048234 | 1 | 1 | 2 | 1 | 0 | 0 | 2 | 9.67 | 21.5 | F | 7 |
| 40045270 | 2 | 1 | 2 | 2 | 1 | 1 | 2 | 24.34 | 21.7 | F | 6 |
| 40047342 | 0 | 1 | 2 | 0 | 2 | 1 | 0 | 8.74 | 22.35 | M | 12 |
| 40042348 | 1 | 1 | 2 | 2 | 1 | 2 | 2 | 24.55 | 23.25 | M | 2.5 |

(continued)

| Genotype and size prediction results for the Mixed 48 set (Example 3) | | | | | | | | | | | |
| Chr | 4 | 10 | 10 | 15 | 20 | 25 | X | | | | |
| SNP ID | 1 | 2 | 3 | 4 | 5 | 6 | 7 | Predicted (kg) | Actual (kg) | Sex | Age (years) |
| 40054426 | 2 | 1 | 1 | 2 | 0 | 0 | 0 | 28.63 | 23.9 | F | 4.75 |
| 40048696 | 0 | 1 | 2 | 2 | 2 | 0 | 0 | 18.70 | 24.2 | F | 2 |
| 40059748 | 2 | 1 | 2 | 1 | 0 | 0 | 1 | 16.96 | 24.3 | F | 5 |
| 40059949 | 2 | 1 | 2 | 2 | 1 | 2 | 0 | 58.50 | 24.4 | F | 2 |
| 40052268 | 2 | 2 | 2 | 1 | 0 | 1 | 2 | 13.76 | 24.85 | M | 2.6 |
| 40037555 | 2 | 1 | 2 | 2 | 2 | 2 | 0 | 52.61 | 25.85 | F | 2.5 |
| 40059825 | 2 | 2 | 2 | 2 | 1 | 2 | 2 | 26.84 | 26.1 | F | 2 |
| 40050617 | 2 | 1 | 2 | 2 | 0 | 2 | 2 | 35.20 | 26.15 | M | 8 |
| 40057568 | 2 | 2 | 2 | 1 | 0 | 1 | 2 | 13.76 | 30.35 | M | 11 |
| 40049706 | 2 | 1 | 2 | 1 | 0 | 0 | 0 | 23.06 | 30.5 | M | 3.6 |
| 40056320 | 2 | 1 | 2 | 2 | 0 | 1 | 1 | 36.8 | 313 | F | 5 |
| 40046852 | 2 | 0 | 2 | 0 | 0 | 2 | 2 | 14.92 | 35 | M | 3.5 |
| 40036517 | 1 | 0 | 2 | 2 | 1 | 1 | 0 | 41.14 | 43.3 | M | 4 |

**Table 7**

| Average allele frequencies for 65 breeds across model SNPs | | | | | | | | |
|---|---|---|---|---|---|---|---|---|
| **Chr** | | **4** | **10** | **10** | **15** | **20** | **25** | **x** |
| **Location** | **Average weight** | **70324248** | **8445140** | **111451490** | **442639801** | **35391970** | **39552390** | **107955905** |
| Mastiff | 82.5 | 0.12 | 0.13 | 0.00 | 1.76 | 0.00 | 1.88 | 0.00 |
| Saint Bernard | 70.5 | 0.40 | 1.47 | 0.00 | 2.00 | 0.13 | 1.87 | 0.00 |
| Newfoundland | 59.0 | 0.52 | 0.16 | 0.00 | 2.00 | 0.16 | 1.52 | 0.00 |
| Great Dane | 50.0 | 0.05 | 0.74 | 0.00 | 2.00 | 1.05 | 2.00 | 0.00 |
| Bullmastiff | 50.0 | 0.05 | 0.60 | 0.00 | 1.90 | 0.00 | 2.00 | 0.00 |
| Irish Wolfhound | 47.5 | 0.58 | 0.00 | 0.00 | 2.00 | 1.70 | 2.00 | 0.00 |
| Alaska Malamute | 47.5 | 0.00 | 0.00 | 0.07 | 2.00 | 0.31 | 0.57 | 0.00 |
| Routtweiler | 45.5 | 0.00 | 1.42 | 0.00 | 0.08 | 0.56 | 1.20 | 0.00 |
| Bloodhound | 43.0 | 0.07 | 2.00 | 0.00 | 2.00 | 1.29 | 1.73 | 0.00 |
| Akita | 42.0 | 0.00 | 1.73 | 0.00 | 2.00 | 0.14 | 1.43 | 0.53 |
| Bernese Mountain Dog | 42.0 | 0.54 | 0.08 | 0.00 | 1.58 | 0.25 | 2.00 | 0.00 |
| Borzoi | 41.5 | 0.23 | 1.23 | 0.00 | 2.00 | 1.38 | 1.85 | 2.00 |
| German Shepherd Dog | 38.5 | 0.47 | 2.00 | 0.00 | 2.00 | 0.57 | 2.00 | 1.07 |
| Dobermann Pinscher | 35.0 | 0.00 | 1.87. | 0.00 | 2.00 | 1.87 | 2.00 | 2.00 |
| Rhodesian Ridgeback | 34.5 | 027 | 1.43 | 0.00 | 1.87 | 0.67 | 1.47 | 0.27 |
| Schnauzer (Giant) | 33.5 | 0.76 | 1.65 | 0.00 | 1.88 | 0.24 | 1.06 | 0.12 |
| Italian Spinone | 32.5 | 0.00 | 1.07 | 0.00 | 1.47 | 1.73 | 1.73 | 0.27 |
| Clumber Spaniel | 32.5 | 0.58 | 1.50 | 0.00 | 2.00 | 2.00 | 0.40 | 0.00 |
| Golden Retriever | 31.5 | 0.08 | 0.77 | 0.00 | 1.69 | 1.38 | 1.38 | 0.00 |
| Labrador Retriever | 29.5 | 0.20 | 0.60 | 0.00 | 1.80 | 1.00 | 1.60 | 0.00 |
| Boxer | 28.5 | 0.07 | 2.00 | 0.00 | 2.00 | 1.71 | 1.20 | 0.00 |
| Belgian Sheepdog | 28.0 | 0.00 | 0.46 | 0.00 | 2.00 | 1.69 | 1.38 | 2.00 |
| Samoyed | 26.5 | 0.00 | 1.73 | 0.00 | 2.00 | 1.87 | 1.87 | 1.73 |

(continued)

Average allele frequencies for 65 breeds across model SNPs

| | Chr | 4 | 10 | 10 | 15 | 20 | 25 | x |
|---|---|---|---|---|---|---|---|---|
| Poodle (Standard) | 26.0 | 0.35 | 0.47 | 0.00 | 1.41 | 1.29 | 1.18 | 1.29 |
| Bull Terrier | 26.0 | 0.00 | 0.00 | 0.00 | 0.46 | 0.00 | 2.00 | 0.00 |
| Afghan Hound | 25.0 | 0.00 | 1.07 | 0.00 | 2.00 | 1.20 | 0.13 | 1.87 |
| Collie (rough) | 24.0 | 0.00 | 2.00 | 0.00 | 2.00 | 0.80 | 1.80 | 2.00 |
| Collie (smooth) | 24.0 | 0.11 | 2.00 | 0.00 | 2.00 | 1.11 | 2.00 | 2.00 |
| Bulldog | 24.0 | 0.00 | 1.20 | 0.00 | 0.53 | 1.85 | 120 | 0.00 |
| English Springer Spaniel | 23.0 | 0.07 | 0.00 | 0.00 | 0.67 | 0.53 | 0.93 | 0.00 |
| Basset Hound | 22.5 | 0.44 | 2.00 | 0.00 | 2.00 | 1.29 | 0.88 | 0.50 |
| Airedale terrier | 21.5 | 0.00 | 2.00 | 0.00 | 1.47 | 2.00 | 2.00 | 0.27 |
| Siberian Huskey | 21.5 | 121 | 1.16 | 0.16 | 2.00 | 0.42 | 0.00 | 0.32 |
| Portuguese Water Dog | 20.5 | 0.13 | 1.41 | 0.00 | 1.50 | 2.00 | 0.50 | 0.25 |
| Saluki | 19.5 | 0.40 | 1.00 | 0.00 | 2.00 | 0.30 | 1.10 | 1.30 |
| Bassett Griffon vendeen (Petit) | 16.0 | 0.31 | 2.00 | 0.00 | 1.85 | 1.08 | 0.46 | 1.38 |
| Schnauzer (Standard) | 15.0 | 0.00 | 1.33 | 0.00 | 0.83 | 0.17 | 0.00 | 0.67 |
| Poodle Miniature | 13.0 | 0.80 | 1.80 | 1.80 | 0.00 | 1.80 | 1.60 | 0.20 |
| American Cocker Spaniel | 12.0 | 1.60 | 1.60 | 0.00 | 0.40 | 1.33 | 2.00 | 0.27 |
| French Bulldog | 11.5 | 0.07 | 0.40 | 0.00 | 0.00 | 0.29 | 1.07 | 0.00 |
| Beagle | 11.0 | 0.27 | 1.47 | 0.27 | 0.13 | 0.46 | 0.14 | 0.29 |
| Pembroke Welsh Corgi | 11.0 | 1.44 | 1.47 | 0.00 | 1.89 | 1.89 | 1.47 | 1.89 |
| Dandie Dinmont Terrier | 9.5 | 1.67 | 2.00 | 0.00 | 2.00 | 2.00 | 0.67 | 0.33 |
| Dachshund LH | 9.0 | 1.79 | 1.29 | 0.00 | 1.71 | 1.86 | 1.43 | 2.00 |
| Dachshund SH | 9.0 | 0.88 | 0.88 | 0.06 | 0.63 | 1.25 | 1.25 | 1.63 |
| Dachshund WH | 9.0 | 0.38 | 0.92 | 0.00 | 0.67 | 1.54 | 0.92 | 1.08 |
| West Highland white terrier | 8.5 | 1.55 | 1.82 | 1.36 | 1.09 | 2.00 | 0.55 | 2.00 |

(continued)

| Chr | | 4 | 10 | 10 | 15 | 20 | 25 | x |
|---|---|---|---|---|---|---|---|---|
| Boston Terrier | 8.0 | 1.71 | 1.00 | 0.00 | 0.31 | 0.71 | 0.71 | 0.71 |
| Manchester Terrier | 7.5 | 036 | 2.00 | 1.64 | 0.00 | 1.82 | 2.00 | 2.00 |
| Pug | 7.0 | 1.27 | 1.47 | 2.00 | 0.00 | 1.20 | 0.67 | 2.00 |
| Parson Russell Terrier | 6.5 | 0.40 | 2.00 | 1.93 | 0.00 | 0.80 | 1.47 | 1.20 |
| Schnauzer (Miniature) | 6.5 | 1.44 | 1.86 | 0.50 | 0.00 | 1.87 | 0.13 | 0.13 |
| Shih Tzu | 6.0 | 0.70 | 1.00 | 2.00 | 0.00 | 1.00 | 1.60 | 1.40 |
| Norwich Terrier | 5.3 | 0.50 | 2.00 | 2.00 | 0.33 | 1.17 | 0.00 | 2.00 |
| Norfolk Terrier | 53 | 0.92 | 2.00 | 2.00 | 2.00 | 1.17 | 0.00 | 2.00 |
| Miniature Pinscher | 4.5 | 0.85 | 1.90 | 1.67 | 0.00 | 1.90 | 1.24 | 0.67 |
| Pekingese | 4.5 | 1.43 | 2.00 | 2.00 | 0.57 | 1.71 | 0.86 | 0.86 |
| Papillon | 4.3 | 0.80 | 1.70 | 0.40 | 0.20 | 1.80 | 1.10 | 2.00 |
| Manchester Terrier (toy) | 4.0 | 1.67 | 2.00 | 2.00 | 0.00 | 2.00 | 1.60 | 0.93 |
| Japanese Chin | 3.5 | 1.20 | 2.00 | 2.00 | 0.20 | 1.00 | 1.80 | 2.00 |
| Italian Greyhound | 3.3 | 0.25 | 1.83 | 1.75 | 0.83 | 1.00 | 0.17 | 1.83 |
| Yorkshire Terrier | 3.0 | 0.92 | 2.00 | 1.92 | 0.33 | 0.62 | 1.23 | 1.69 |
| Maltese | 2.5 | 0.93 | 1.07 | 1.93 | 0.27 | 2.00 | 0.53 | 1.73 |
| Chihuahua | 2.0 | 0.80 | 2.00 | 1.90 | 0.00 | 1.40 | 1.20 | 1.40 |
| Chihuahua (long coat) | 2.0 | 0.17 | 2.00 | 2.00 | 0.00 | 1.67 | 1.67 | 2.00 |

Average allele frequencies for 65 breeds across model SNPs

**Table 8**

| "Atypical" Breed | IGF1 SNP Average allele frequency | IGF1 SNP Average allele frequency of similar sized breeds | Genotyped SNP result | Genotyped SNP result as a score (0,1 or 2) | Predicted allele of second breed | Predicted allele of "atypical" breed | Modified allele of "atypical" breed | Modified genotype | Modified genotype as a score (0,1 or 2) |
|---|---|---|---|---|---|---|---|---|---|
| A conversion matrix for atypical breeds at IGF1 SNP | | | | | | | | | |
| Rottweiler | 0.08 | 2 | AA | 0 | A | A | a | Aa | 1 |
| | | | Aa | 1 | A | a | a | Aa | 1 |
| | | | aA | 1 | a | A | a | aa | 2 |
| | | | aa | 2 | a | a | a | aa | 2 |
| Bull terrier | 0.46 | 2 | AA | 0 | A | A | a | Aa | 1 |
| | | | Aa | 1 | A | a | a | Aa | 1 |
| | | | aA | 1 | a | A | a | aa | 2 |
| | | | aa | 2 | a | a | a | aa | 2 |
| Whippet | 1.89 | 0 | AA | 0 | A | A | A | AA | 0 |
| | | | Aa | 1 | A | a | A | AA | 0 |
| | | | aA | 1 | a | A | A | aA | 1 |
| | | | aa | 2 | a | a | A | aA | 1 |

EP 2 212 436 B1

33

## Table 9

Chromosome 15 breed calls for Mixed 48 Set

| | Chr15 (breed 1) | Chr15 (breed 2) |
|---|---|---|
| 40036517 | Fox Terrier (Wire) | Labrador Retriever^2 |
| 40036518 | Shih Tzu | Cavalier King Charles Spaniel |
| 40036652 | Manchester Terrier | Staffordshire Bull Terrier |
| 40037555 | Irish Setter^UK | German Shepherd Dog |
| 40040572 | ? | ? |
| 40042348 | ? | border collie |
| 40043243 | ? | ? |
| 40043711 | ? | ? |
| 40045270 | border collie | greyhound |
| 40046359 | shetland sheepdog | kerry blue terrier |
| 40046852 | Staffordshire Bull Terrier | bullterrier |
| 40046997 | Border collie | English Cocker Spaniel |
| 40047342 | eng cocker spaniel | Poodle (Miniature) |
| 40047491 | Yorkshire terrier | Yorkshire terrier |
| 40047766 | shetland sheep dog | border collie |
| 40047873 | chinese shar pei | shetlanf sheep dog (or stafford shire bull terrier) |
| 40048130 | cavalier king charles spaniel | Shetland Sheepdog |
| 40048234 | german shepherd | English setter |
| 40048252 | german shepherd dog | german shepherd dog |
| 40048696 | English Springer | Labrador Retriever^2 |
| 40049353 | Australian Cattle dog | Parson russell terrier |
| 40049466 | welsh terrier | parson russell terrier |
| 40049706 | cavalier king charles spaniel | Portugese water dog |
| 40049961 | Whippet | Whippet |
| 40049974 | yorkshire terrier | west highland white |
| 40050208 | yorkshire terrier | whippet |
| 40050617 | samoyed | bearded or border collie |
| 40050815 | chinese crested | Yorkshire terrier |
| 40051462 | ? | ? |
| 40052268 | rottweiler | old english sheepdog |
| 40052662 | german shepherd dog | old english sheep dog |
| 40053199 | Parson Jack russell | japenese chin |
| 40054384 | English Springer Spaniel | Border Terrier |
| 40054426 | Boxer | Am staff |
| 40055160 | ? | ? |
| 40055628 | Parson Russel terrier | Toy fox terrier |
| 40055850 | ? | ? |
| 40056320 | german shepherd dog | german shepherd dog |
| 40056389 | Border Collie | Labrador Retriever |
| 40056463 | ? | ? |
| 40057568 | Border collie | papillion |
| 40058848 | ? | ? |
| 40059059 | cocker spaniel | ? |
| 40059748 | greyhound | border collie |
| 40059825 | german shepherd dog | german shepherd dog |
| 40059930 | shetland sheep dog | yorkshire terrier |
| 40059949 | poodle | boxer |
| 40060406 | lhasa apso | king charles cavalier spaniel |

**Table 10**

| Table showing the effects of the modification matrix when applied to the Mixed 48 set | | | | | | | |
|---|---|---|---|---|---|---|---|
| | Before modification | | | | | | |
| | 4 | 10 | 10 | 15 | 20 | 25 | X |
| | BICFPJ1149345 | BICF230J87378 | BICF235J47583 | BICFPJ401056 | BICF235J20169 | BICF235J29129 | BICF235J47857 |
| 40048852 | 2 | 0 | 2 | 0 | 0 | 2 | 2 |
| 40049981 | 2 | 0 | 2 | 2 | 0 | 2 | 2 |
| 40050208 | 2 | 2 | 1 | 0 | 1 | 2 | 2 |
| 40052268 | 2 | 2 | 2 | 1 | 0 | 1 | 2 |
| | | | | | | | |
| | After modification | | | | | | |
| | 4 | 10 | 10 | 15 | 20 | 25 | X |
| | B1CFPJ1149345 | BICF230J67378 | BICF235J47583 | BICFPJ401056 | BICF235J20169 | BICF235J29129 | BICF235J47857 |
| 40046852 | 2 | 0 | 2 | 1 | 0 | 2 | 2 |
| 40049961 | 2 | 0 | 2 | 0 | 0 | 2 | 2 |
| 40050208 | 2 | 2 | 1 | 0 | 1 | 2 | 2 |
| 40052268 | 2 | 2 | 2 | 2 | 0 | 1 | 2 |
| | | | | | | | |
| | Standard predicted | Modified predicted | Actual wt | | | | |
| 4006852 | 14.92 | 24.89 | 35 | | | | |
| 40049861 | 41.52 | 14.92 | 10.98 | | | | |
| 40050208 | 7.18 | 7.18 | 10.6 | | | | |
| 40052268 | 13.76 | 22.95 | 24.85 | | | | |

**Claims**

1. A method of predicting the size of a dog that will be attained in adulthood, comprising typing the nucleotides present for the combination of single nucleotide polymorphic (SNP) markers present in the genome of the dog at positions equivalent to position 201 of the following sequences:

   - SEQ ID NO: 58 (BICF235J47583, SNP 3);
   - SEQ ID NO: 84 (BICFPJ401056, SNP 4);
   - SEQ ID NO: 146 (BICF235J47857, SNP 7);
   - SEQ ID NO: 7 (BICFPJ1149345, SNP 1);
   - SEQ ID NO: 35 (BICF230J67378, SNP 2);
   - SEQ ID NO: 96 (BICF235J20169, SNP 5); and
   - SEQ ID NO: 111 (BICF235J29129, SNP 6) and thereby predicting the size of the dog that will be attained in adulthood.

2. The method according to claim 1, wherein the size predicted is selected from the weight or height of the dog.

3. The method according to claims 1 or 2, further comprising inputting the results into a model for predicting the size of the dog, wherein the size of the dog is predicted by adding a multiplication of the value of one SNP marker by a constant and adding a second constant, wherein the result is the log-body weight of the dog in kg.

4. The method of claim 3, further comprising adding a multiplication of the value of a different SNP marker by a constant.

5. The method according to claim 4, comprising adding a multiplication of the value of a SNP marker by a constant for each of the SNP markers defined in claim 1 and adding a second constant.

6. The method according to any one of the preceding claims, further comprising determining the genetic breed inheritance of the dog.

7. The method according to any one of the preceding claims, wherein:

   (i) the nucleotide present at a polymorphic position is detected by measuring the mobility of a polynucleotide during gel electrophoresis; or
   (ii) the nucleotide present at a polymorphic position is determined by hybridising at least one oligonucleotide primer and contacting the sample with a polymerase under conditions suitable for generation of a primer extension product, wherein determining the nucleotide comprises detecting the presence of the primer extension product.

8. The method according to any one of the preceding claims, further comprising providing the dog's owner or carer with a report of the predicted size of the dog that will be attained at adulthood.

9. The method according to claim 8, further comprising providing care recommendations to the dog's owner or carer to control the weight or growth rate of the dog.

10. A method of predicting the size of a dog that will be attained in adulthood, the method comprising:

    (a) inputting data of the nucleotides, and optionally the breed origin of the nucleotides, present at the SNP marker positions in the dog's genome as defined in claim 1 to a computer system;
    (b) comparing the data to a computer database, which database comprises information relating to the polymorphisms as defined in claim 1 and their association with the size of a dog in adulthood; and
    (c) predicting on the basis of the comparison the size of the dog that will be attained in adulthood.

11. Use of the combination of SNP markers present in the genome of a dog as defined in claim 1 for predicting the size that a dog will attain in adulthood.

**Patentansprüche**

1. Verfahren zur Vorhersage der Größe eines Hundes, die er im Erwachsenenalter erreichen wird, umfassend das Typisieren der Nukleotide, die für die Kombination von Einzelnukleotidpolymorphismus-(SNP)-Markern vorhanden sind, welche im Genom des Hundes an Positionen vorliegen, die der Position 201 der folgenden Sequenzen entsprechen:

   - SEQ ID NO: 58 (BICF235J47583, SNP 3);
   - SEQ ID NO: 84 (BICFPJ401056, SNP 4);
   - SEQ ID NO: 146 (BICF235J47857, SNP 7);
   - SEQ ID NO: 7 (BICFPJ1149345, SNP 1);
   - SEQ ID NO: 35 (BICF230J67378, SNP 2);
   - SEQ ID NO: 96 (BICF235J20169, SNP 5); und
   - SEQ ID NO: 111 (BICF235J29129, SNP 6),

   und dadurch Vorhersagen der Größe des Hundes, die er im Erwachsenenalter erreichen wird.

2. Verfahren nach Anspruch 1, wobei die vorhergesagte Größe aus dem Gewicht oder der Körpergröße des Hundes ausgewählt wird.

3. Verfahren nach Anspruch 1 oder 2, ferner das Eingeben der Ergebnisse in ein Modell zur Vorhersage der Größe des Hundes umfassend, wobei die Größe des Hundes durch Addieren eines Produktes aus dem Wert eines SNP-Markers und einer Konstante und Addieren einer zweiten Konstante vorhergesagt wird, wobei das Ergebnis der Logarithmus des Körpergewichts des Hundes in kg ist.

4. Verfahren nach Anspruch 3, ferner das Addieren eines Produkts aus dem Wert eines anderen SNP-Markers und einer Konstante umfassend.

5. Verfahren nach Anspruch 4, das Addieren eines Produkts aus dem Wert eines SNP-Markers und einer Konstanten für jeden SNP-Marker, der in Anspruch 1 definiert ist, und das Addieren einer zweiten Konstante umfassend.

6. Verfahren nach einem der vorhergehenden Ansprüche, ferner das Bestimmen des genetischen Erbguts der Rasse des Hundes umfassend.

7. Verfahren nach einem der vorhergehenden Ansprüche, wobei:

   (I) das an einer polymorphen Position vorhandene Nukleotid durch Messen der Mobilität eines Polynukleotids während einer Gelelektrophorese detektiert wird; oder
   (II) das an einer polymorphen Position vorhandene Nukleotid durch Hybridisieren mindestens eines Oligonukleotid-Primers und Inkontaktbringen der Probe mit einer Polymerase unter Bedingungen, die zur Erzeugung eines Primer-Extensions-Produktes geeignet sind, bestimmt wird, wobei das Bestimmen des Nukleotids das Detektieren des Vorhandenseins des Primer-Extensions-Produktes umfasst.

8. Verfahren nach einem der vorhergehenden Ansprüche, ferner das Bereitstellen eines Berichts über die vorhergesagte Größe des Hundes, die er im Erwachsenenalter erreichen wird, für den Hundehalter oder -pfleger umfassend.

9. Verfahren nach Anspruch 8, ferner das Bereitstellen von Pflegeempfehlungen zur Kontrolle des Gewichts oder der Wachstumsrate des Hundes für den Hundehalter oder - pfleger umfassend.

10. Verfahren zur Vorhersage der Größe eines Hundes, die er im Erwachsenenalter erreichen wird, wobei das Verfahren Folgendes umfasst:

   (a) Eingeben von Daten der Nukleotide und optional des Rassenursprungs der in Anspruch 1 definierten Nukleotide, die an SNP-Marker-Positionen im Genom des Hundes vorhanden sind, in ein Computersystem,;
   (b) Vergleichen der Daten mit einer Computer-Datenbank, wobei die Datenbank Informationen umfasst, die den in Anspruch 1 definierten Polymorphismus und dessen Verbindung mit der Größe eines Hundes im Erwachsenenalter betreffen; und
   (c) Vorhersagen der Größe des Hundes, die er im Erwachsenenalter erreichte wird, auf der Grundlage des Vergleichs.

11. Verwendung der Kombination von in Anspruch 1 definierten SNP-Markern, die in einem Genom eines Hundes vorhanden sind, zum Vorhersagen der Größe des Hundes, die er im Erwachsenenalter erreichen wird.

**Revendications**

1. Procédé permettant de prédire la taille d'un chien qui sera atteinte à l'âge adulte, comprenant la saisie des nucléotides présents pour la combinaison de marqueurs polymorphes nucléotidiques simples (SNP) présents dans le génome du chien à des positions équivalentes à la position 201 des séquences suivantes :

   - SEQ ID n° : 58 (B1CF235J47583, SNP 3) ;
   - SEQ ID n° : 84 (B1CFPJ401056, SNP 4) ;
   - SEQ ID n° : 146 (B1CF235J47857, SNP 7) ;
   - SEQ ID n° : 7 (B1CFPJ1149345, SNP 1) ;
   - SEQ ID n° : 35 (B1CF230J67378, SNP 2) ;
   - SEQ ID n° : 96 (B1CF235J20169, SNP 5) ; et
   - SEQ ID n° : 111 (B1CF235J29129, SNP 6) ;

   et ainsi la prédiction de la taille du chien qui sera atteinte à l'âge adulte.

2. Procédé selon la revendication 1, dans lequel la taille prédite est choisie parmi le poids ou la hauteur du chien.

3. Procédé selon la revendication 1 ou 2, comprenant en outre l'entrée des résultats dans un modèle destiné à prédire la taille du chien, ladite taille du chien étant prédite par l'addition d'une multiplication de la valeur d'un marqueur SNP par une constante et par l'addition d'une deuxième constante, le résultat étant le poids corporel logarithmique du chien en kg.

4. Procédé selon la revendication 3, comprenant en outre l'addition d'une multiplication de la valeur d'un marqueur SNP différent par une constante.

5. Procédé selon la revendication 4, comprenant l'addition d'une multiplication de la valeur d'un marqueur SNP par une constante pour chacun des marqueurs SNP définis dans la revendication 1 et l'addition d'une deuxième constante.

6. Procédé selon l'une quelconque des revendications précédentes, comprenant en outre la détermination du patrimoine génétique du chien.

7. Procédé selon l'une quelconque des revendications précédentes, dans lequel :

   (i) le nucléotide présent à une position polymorphe est détecté par la mesure de la mobilité d'un polynucléotide pendant l'électrophorèse sur gel ; ou
   (ii) le nucléotide présent à une position polymorphe est déterminé par l'hybridation d'au moins une amorce oligonucléotidique et la mise en contact de l'échantillon avec une polymérase dans des conditions permettant de générer un produit d'extension d'amorce, la détermination du nucléotide comprenant la détection de la présence du produit d'extension d'amorce.

8. Procédé selon l'une quelconque des revendications précédentes, comprenant en outre la remise au propriétaire du chien ou à la personne s'occupant du chien d'un rapport sur la taille prédite du chien qui sera atteinte à l'âge adulte.

9. Procédé selon la revendication 8, comprenant en outre la dispense de conseils de soins au propriétaire ou à la personne s'occupant du chien pour contrôler la prise de poids ou le taux de croissance du chien.

10. Procédé permettant de prédire la taille d'un chien qui sera atteinte à l'âge adulte, ledit procédé comprenant :

   (a) l'entrée, dans un système informatique, de données sur les nucléotides, et éventuellement l'origine de la race des nucléotides, présents aux positions des marqueurs SNP dans le génome du chien tel que défini dans la revendication 1 ;
(b) la comparaison des données à une base de données informatique, ladite base de données comprenant des informations relatives aux polymorphismes tels que définis dans la revendication 1 et leur association à la taille d'un chien à l'âge adulte ; et
(c) la prédiction en fonction de la comparaison de la taille du chien qui sera atteinte à l'âge adulte.

11. Utilisation de la combinaison de marqueurs SNP présents dans le génome d'un chien tel que défini dans la revendication 1 permettant de prédire la taille qu'un chien atteindra à l'âge adulte.

**Figure 1**

Figure 2

**Figure 3**

## Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SUTTER et al.** *Science,* 2007, vol. 316, 112-115
- **BRUCE FOGEL.** The Encyclopaedia of the dog. Dorling Kindersley, 2000
- *PCR Methods and Applications,* 1994, vol. 3, 268-71
- *Proc. Natl. Acad. Sci.,* 1998, vol. 85, 4397-4401
- **DEVEREUX et al.** *Nucleic Acids Research,* 1984, vol. 12, 387-395
- **ALTSCHUL S. F.** *J Mol Evol,* 1993, vol. 36, 290-300
- **ALTSCHUL, S, F et al.** *J Mol Biol,* 1990, vol. 215, 403-10
- **HENIKOFF.** *Proc. Natl. Acad. Sci. USA,* 1992, vol. 89, 10915-10919
- **KARLIN ; ALTSCHUL.** *Proc. Natl. Acad. Sci.,* 1993, vol. 90, 5873-5787